# EUROPEAN PATENT APPLICATION

(11) **EP 2 592 154 A1**
(43) Date of publication of application: **15.05.2013**
(21) Application number: 11008923.2
(22) Date of filing: 09.11.2011
(51) Int. Cl.: C12Q 1/34, G01N 33/68, C12Q 1/48, G01N 33/573, C07D 401/14, C07D 401/04, C12N 11/06

(54) **Immobilization products and methods for the identification of histone demethylase interacting molecules and for the purification of histone demethylase proteins**

(71) Applicant: CELLZOME AG, 69117 Heidelberg (DE); Glaxo Group Limited, Middlesex UB6 0NN (GB)
(72) Inventor: Atkinson, Stephen John, Stevenage, Hertfordshire SG1 2NY (GB); Barker, Michael David, Stevenage, Hertfordshire SG1 2NY (GB); Campbell, Matthew, Stevenage, Hertfordshire SG1 2NY (GB); Humphreys, Philip, Stevenage, Hertfordshire SG1 2NY (GB); Liddle, John, Stevenage, Hertfordshire SG1 2 NY (GB); Sheppard, Robert John, Stevenage, Hertfordshire SG1 2 NY (GB); Wilson, David, Stevenage, Hertfordshire SG1 2 NY (GB); Joberty, Gerard, 69118 Heidelberg (DE)
(74) Representative: Lahrtz, Fritz

(57) **Abstract**

The present invention relates to immobilization products comprising compounds of formula (I) and preparations thereof as well as methods and uses for the identification of histone demethylase interacting compounds or for the purification or identification of histone demethylase proteins.

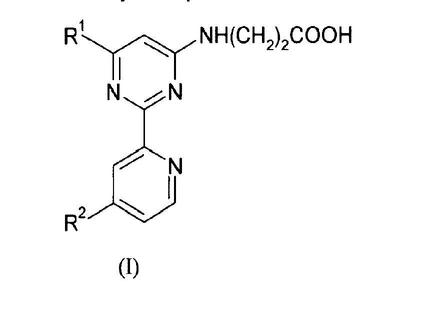

## Description

The present invention relates to immobilization products and preparations thereof as well as methods and uses for the identification of histone demethylase interacting compounds or for the purification or identification of histone demethylase proteins.

Epigenetic information refers to heritable changes in gene function that are stable between cell divisions but without changing the DNA sequence. Part of the epigenetic mechanism has been ascribed to modifications of histone proteins or DNA that affects the expression of specific genes. The post-translational modifications of histone tails such as the methylation of arginine and lysine amino acid residues are important for the storage of epigenetic information (Agger et al., 2008. Curr. Opin. Genet. Dev. 18(2):159-168).

The histone-modifying enzymes that catalyse the demethylation of lysine residues (lysine demethylases, KDMs) and arginine residues (arginine demethylases, RDMs) are of substantial interest from the perspective of drug discovery and medicinal chemistry. These enzymes play important roles in controlling gene regulation, and there is evidence that the enzymatic activities of several of these proteins have pathogenic roles in for example in diseases such as cancer (Kampranis and Tsichlis, 2009. Adv. Cancer Res. 102:103-169).

Of particular interest are lysine demethylases of the Jumonji family which as a common feature share the catalytic Jumonji C (JmjC) protein domain. There are 27 different human JmjC domain proteins of which 15 have been shown to demethylate specific lysines in the histone 3 (H3) tail and one to demethylate methylated arginine. The catalytic JmjC domain is essential for the oxidative lysine demethylation reaction that requires Fe(II) and α-ketoglutarate (αKG) as cofactors (Agger et al., 2008. Curr. Opin. Genet. Dev. 18(2):159-168).

For the development of drugs targeting histone demethylases it is important to have assays that allow the identification and characterization of small molecule inhibitors in terms of potency and selectivity across the histone demethylase family (Hamada et al, 2010. J. Med. Chem. 53(15):5629-5638).

Therefore another step for the identification of selective histone demethylase inhibitors is a method that allows to determine the target selectivity of these molecules. For example, it can be intended to provide molecules that bind to and inhibit a particular drug target but do not interact with a closely related target, inhibition of which could lead to unwanted side effects. Conventionally panels of individual enzyme assays are used to assess the inhibitory effect of a compound for protein histone demethylases (Hamada et al, 2010. J. Med. Chem. 53(15):5629-5638).

The *in vitro* investigation of histone demethylase activity is typically performed using enzyme assays. For example, the recombinant histone demethylases GASC1, JMJD2a and JMJD2b were expressed using baculovirus vectors in insect cells and tested in demethylation assays (Cloos et al., 2006. Nature 442(7100):307-311). However, it can be advantageous to use endogenous histone demethylases as isolated from mammalian cells.

Several reports described histone demethylase inhibitors (Hamada et al., 2009. Bioorg Med Chem Lett. 19(10):2852-2855; Hamada et al, 2010. J. Med. Chem. 53(15):5629-5638; Rose et al., 2010. J. Med. Chem. 53(4):1810-1818; WO2010043866A3).

In view of the above, there is a need for providing effective tools and methods for the identification and selectivity profiling of histone demethylase interacting compounds as well as for the purification of histone demethylases.

The present invention relates inter alia to an immobilization product comprising a compound of formula (I) 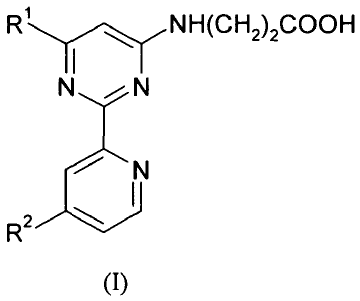 wherein

R¹ is:
- C₃₋₇ cycloalkyl;
- C₁₋₆ haloalkyl;
- a 5, 6 or 7-membered aryl or heteroaryl (which heteroaryl contains one or more heteroatoms selected from N, O and S and which is optionally fused to phenyl), said 5, 6 or 7-membered aryl or heteroaryl being optionally substituted with one or more substituents independently selected from C₁₋₃alkyl;
- O-C₁₋₆alkyl (which is optionally substituted by phenyl);
- O-cyclohexyl (which is optionally fused with phenyl);
- C(O)NR^{c}₂;
   or
- NR^{a}R^{b},
   each R^{a} and R^{b} is independently selected from:
- H;
- C₁₋₃alkyl which is optionally substituted by one or more substituents independently selected from phenyl (which phenyl is optionally substituted by one or more substituents independently selected from C₁₋₃alkyl, O-C₁₋₃alkyl, C(O)NR^{c}₂, halo and cyano), C(O)NR^{c}₂, a 4, 5, 6 or 7-membered heterocyclic or heteroaryl group (containing one or more heteroatoms independently selected from N, O and, S), a 3, 4, 5, 6 or 7-membered cycloalkyl group (which is optionally fused to phenyl), halo, OC₁₋₃alkyl, OH, -NHCOC₁₋₃alkylNR^{c}₂ and C(O)NHCH₂C(O)NR^{c}₂;
- a 3, 4, 5, 6 or 7-membered cycloalkyl group (which is optionally fused to phenyl),
   or
- R^{a} and R^{b} together form a 5, 6 or 7-membered heterocyclic group optionally containing one or more further heteroatoms independently selected from N, O, S or S(O)₂ said heterocyclic group being optionally fused to a 5, 6 or 7-membered aryl or heteroaryl ring containing one or more heteroatoms independently selected from N, O and S; the heterocylic ring and/or the aryl or heteroaryl to which it is optionally fused being optionally substituted by one or more substituents independently selected from halo, OH, C₁₋₃alkyl, O-C₁₋₃alkyl, C(O)C₁₋₃alkyl, S(O)₂C₁₋₃alkyl, NHC(O)C₁₋₃alkyl, NHS(O)₂C₁₋₃alkyl, C(O)NR^{c}₂, C(O)NR^{d}₂ (wherein R^{d} and R^{d} together form a 5 or 6-membered heterocylic ring), NR^{c}₂, C(O)phenyl, S(O)₂NR^{c}₂, =O (oxo) and 5, 6 or 7-membered aryl or heteroaryl (containing one or more heteroatoms independently selected from N, O and S);

R² is:
- (CH₂)₁₋₃NR^{c}(CH₂)₁₋₃NHR^{c},
- (CH₂)₁₋₆NHR^{c};
- (CH₂)₀₋₃NHR^{a} (wherein R^{a} is as defined above); or
- (CH₂)₀₋₃NHPh;
and
each R^{c} is independently selected from hydrogen and C₁₋₃alkyl,
or a salt thereof characterized in that the compound of formula (I) is immobilized on a solid support.

In case a variable or substituent can be selected from a group of different variants and such variable or substituent occurs more than once the respective variants can be the same or different.

Within the meaning of the present invention the terms are used as follows:
As used herein, the term "alkyl" and "alkylene" refers to a saturated hydrocarbon chain having the specified number of member atoms. C₁₋₆alkyl refers to an alkyl group having from 1 to 6 member atoms, for example 1 to 4 member atoms. Alkyl groups may be straight or branched. Representative branched alkyl groups have one, two, or three branches. Alkyl includes methyl, ethyl, propyl (n-propyl and isopropyl), butyl (n-butyl, isobutyl, and t-butyl), pentyl (n-pentyl, isopentyl, and neopentyl), and hexyl.

As used herein, the term "halo" refers to the halogen radical fluoro, chloro, bromo, or iodo.

As used herein, the terms "C₁-C₆ haloalkyl", "C₁₋₆haloalkyl" and the like refer to a straight or branched chain alkyl group as defined above containing at least 1, and at most 6 carbon atoms respectively substituted with at least one halo group, halo being as defined herein. Examples of such branched or straight chained haloalkyl groups useful in the present invention include, but are not limited to, methyl, ethyl, propyl, isopropyl, isobutyl and n-butyl substituted independently with one or more halos, e.g., fluoro, chloro, bromo and iodo.

As used herein, the term "heterocyclic group" refers to a non-aromatic ring having the specified number of member atoms being saturated or having one or more degrees of unsaturation and, unless otherwise specified, containing one or more heteroatoms selected from N, O or S.

As used herein, the term "aryl" refers to an aromatic ring having the specified number of member atoms. Bicyclic and other polycyclic ring systems containing an aryl are described as fused systems.

As used herein, the term "heteroaryl" refers to an aromatic ring having the specified number of member atoms and, unless otherwise specified, containing one or more heteroatoms selected from N, O or S. Bicyclic and other polycyclic ring systems comprising a heteroaryl ring are described as fused systems.

As used herein, the term "member atoms" refers to the atom or atoms that form a chain or ring. Where more than one member atom is present in a chain and within a ring, each member atom is covalently bound to an adjacent member atom in the chain or ring. Atoms that make up a substituent group on a chain or ring are not member atoms in the chain or ring.

As used herein, the term "optionally substituted" indicates that a group may be unsubstituted or substituted with one or more substituents as defined herein.

As used herein, the term "substituted" in reference to a group indicates that a hydrogen atom attached to a member atom within a group is replaced. It should be understood that the term "substituted" includes the implicit provision that such substitution be in accordance with the permitted valence of the substituted atom and the substituent and that the substitution results in a stable compound (i.e. one that does not spontaneously undergo transformation such as by rearrangement, cyclization, or elimination). In certain embodiments, a single atom may be substituted with more than one substituent as long as such substitution is in accordance with the permitted valence of the atom. Suitable substituents are defined herein for each substituted or optionally substituted group.

Preferred compounds of formula (I) are those compounds in which one or more of the residues contained therein have the meanings given below, with all combinations of preferred substituent definitions being a subject of the present invention. With respect to all preferred compounds of the formula (I), the present invention also includes all tautomeric and stereoisomeric forms and mixtures thereof in all ratios, and their pharmaceutically acceptable salts.

In preferred embodiments of the present invention, the substituents mentioned below independently have the following meaning. Hence, one or more of these substituents can have the preferred or more preferred meanings given below.

Preferably, R¹ is NR^{a}R^{b}; with R^{a} and R^{b} as defined above.

More preferably
R¹ is NHCH₂Ph, 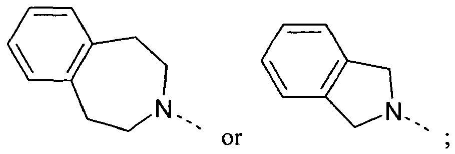 and R² is (CH₂)₀₋₃NHPh or (CH₂)₁₋₆NHR^{c,} with R^{c} as defined above.

Compounds of formula (I) in which some or all of the above-mentioned groups have the preferred meanings are also an object of the present invention.

In case the compounds according to formula (I) contain one or more acidic or basic groups, the invention also comprises their corresponding salts. Thus, the compounds of the formula (I) which contain acidic groups can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Compounds of the formula (I) which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the formula (I) simultaneously contain acidic and basic groups in the molecule, the invention also includes, in addition to the salt forms mentioned, inner salts or betaines (zwitterions). The respective salts according to the formula (I) can be obtained by customary methods which are known to the person skilled in the art like, for example by contacting these with an organic or inorganic acid or base in a solvent or dispersant, or by anion exchange or cation exchange with other salts.

A preferred compound of formula (I) is 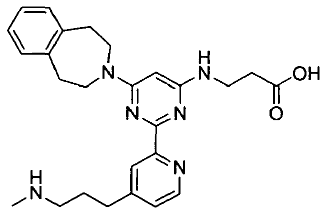 3-((6-(4,5-dihydro-1H-benzo[d]azepin-3(2H)-yl)-2-(4-(3-(methylamino)propyl)pyridin-2-yl)pyrimidin-4-yl)amino)propanoic acid.

Further preferred compounds of the present invention can be selected from the group consisting of:
N-{2-{4-[3-(methylamino)propyl]-2-pyridinyl}-6-[(phenylmethyl)amino]-4-pyrimidinyl}-β-alanine
N-{2-(4-{3-[(2-aminoethyl)amino]propyl}-2-pyridinyl)-6-[(phenylmethyl)amino]-4-pyrimidinyl}-β-alanine
N-(6-(1,3-dihydro-2H-isoindol-2-yl)-2-{4-[(phenylamino)methyl]-2-pyridinyl}-4-pyrimidinyl)-β-alanine

The compounds of the present invention can be prepared by methods well known in the art. Exemplary routes for the preparation of compounds of the present invention are described below. It is clear to a practitioner in the art to combine or adjust such routes especially in combination with the introduction of activating or protective chemical groups.

Compounds of formula (I) may be prepared by one or more of the following general schemes, and the routes described in the specific Examples section below.

**Scheme 1 -** Synthesis of *N*-[6-alkyl-2-(2-pyridinyl)-4-pyrimidinyl]-β-alanines, *N*-[6-amide-2-(2-pyridinyl)-4-pyrimidinyl]-β-alanines and *N*-[6-alkyl amino-2-(2-pyridinyl)-4-pyrimidinyl]-β-alanines 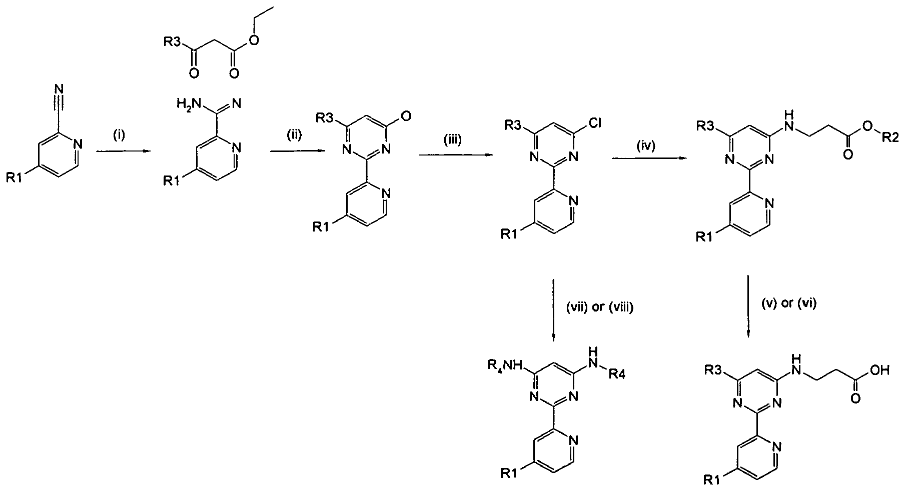
(i) NaOMe, ammonium chloride, EtOH
(ii) Sodium ethoxide in EtOH, Reflux or Na in EtOH, Reflux
(iii) POCl₃
(iv) Ethyl or *t*-butyl *β*-alaninate hydrochloride, DIPEA, 1,4-Dioxane, microwave
(v) Where R2 = Me or Et, LiOH, 1:1 THF/H₂O with or without MeOH, or KOH in MeOH, Reflux
(vi) Where R2 = tBu, 5M HCl, THF
(vii) Where R3 = Cl, R4NH₂, DIPEA, DMSO, microwave
(viii) Where R3 = Cl, R4NH₂,NaO*t*Bu, [2'-(dicyclohexylphosphanyl)-2-biphenylyl]dimethylamine, Pd₂(dba)₃, 1,4-dioxane, microwave

**Scheme 2** - Synthesis of *N*-[6-(aryl-)-2-(2-pyridinyl)-4-pyrimidinyl]-*β*-alanines, *N*-[6-(alkyloxy-)-2-(2-pyridinyl)-4-pyrimidinyl]-*β*-alanines, and *N*-[6-(alkylamino-)-2-(2-pyridinyl)-4-pyrimidinyl]-*β*-alanines and their methyl and ethyl esters 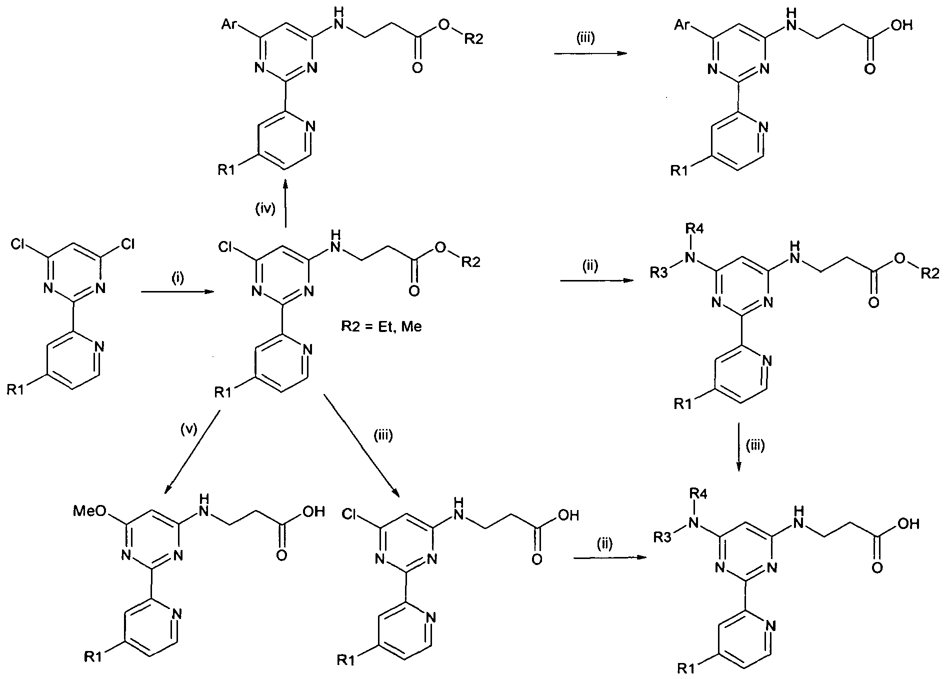
(i) Methyl *β*-alaninate or Ethyl *β*-alaninate hydrochloride, DIPEA, 1,4-Dioxane
(ii) R3R4NH, DIPEA, DMSO, microwave
(iii) LiOH, 1:1 THF/H₂O with or without MeOH
(iv) Aryl boronic acid or aryl boronic acid pinacol ester, Pd(PPh₃)₄,1,4-dioxane/H₂O, microwave
(v) 25% by wt NaOMe in MeOH, DMSO, microwave

**Scheme 3 -** Synthesis of *N*-[6-[(alkyloxy- or *N*-amido-]-2-(2-pyridinyl)-4-pyrimidinyl]-β-alanine 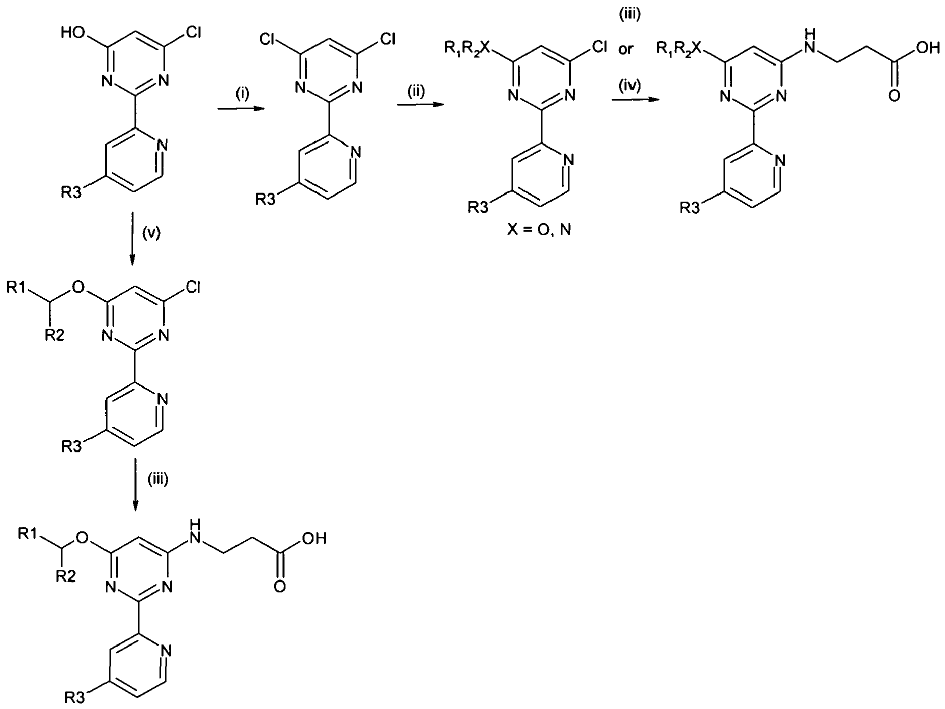
(i) POCl₃
(ii) R1R2OH or R1R2NH, NaH, DMF
(iii) *β*-alanine, DIPEA, 1,4-Dioxane, microwave
(iv) Ethyl *β*-alaninate, DIPEA, 1,4-Dioxane, microwave then LiOH, THF/H₂O
(v) R1R2CHOH, DIAD, PPh₃, THF

**Scheme 4** - Synthesis of *N*-{2-[4-(3-aminopropyl)-2-pyridinyl]-6-[(phenylmethyl)amino]-4-pyrimidinyl}-β-alanine derivatives 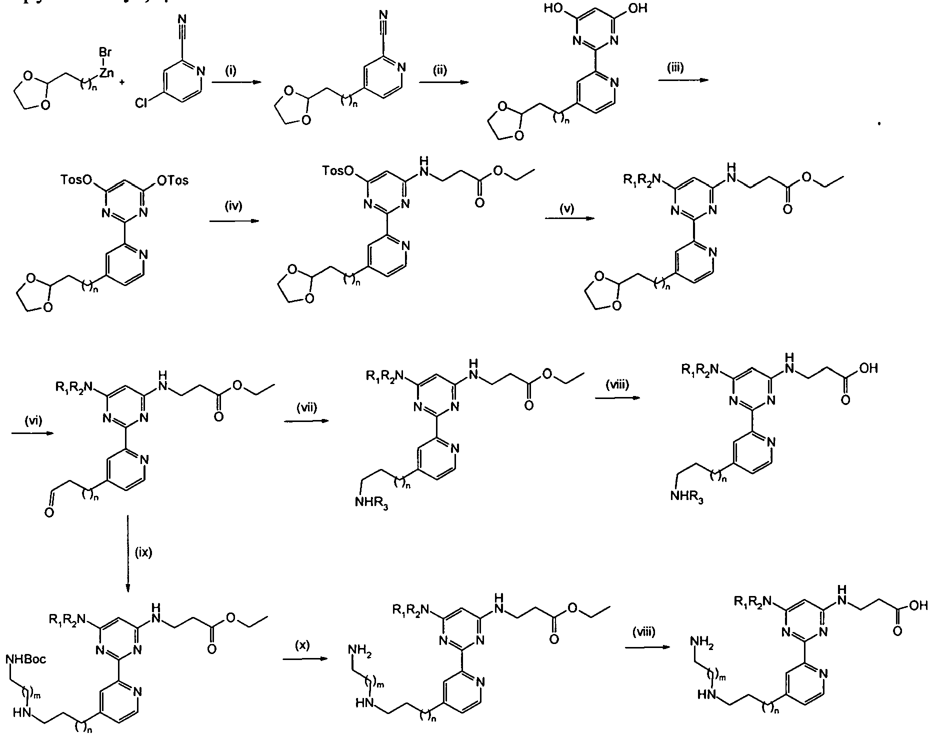
(i) Pd(PPh₃)₄,110°C, microwave, 1h
(ii) NaOMe, MeOH,NH₄Cl then diethyl propanedioate, NaOMe, reflux
(iii) *p*-TosCl, NEt₃, DMAP, DCM
(iv) Ethyl β-alaninate (HCl salt), MeCN, NEt₃,100°C, microwave, 20min
(v) R1R2NH,150°C, microwave, IPA, 1h
(vi) AcOH, H₂O, 80°C
(vii) R3NH₂,NaBH₄,THF
(viii) LiOH, THF/H₂O
(ix) 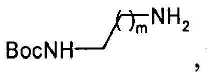 NaBH₄, THF
(x) TFA, DCM

The immobilization products of the invention are e.g. useful in the methods of the invention for the identification of histone demethylase interacting compounds or in diagnostic methods for the diagnosis of inflammatory diseases, proliferative diseases and metabolic diseases.

Throughout the invention, the term "solid support" relates to every undissolved support being able to immobilize a small molecule ligand on its surface.

According to the invention, the term "immobilization product" means either that at least one compound of the same type is immobilized on the solid support or that one or more different compounds (each of them either in singular or plural) may be immobilized on the solid support. Preferably, one or two different compounds are immobilized on the solid support, more preferably the preferred compounds of formula (I).

In another preferred embodiment, the preferred compound of formula (I) 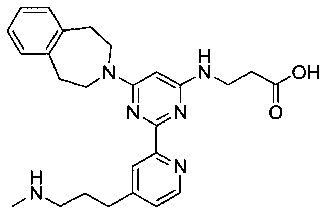 is immobilized.

The solid support may be selected from the group consisting of agarose, modified agarose, sepharose beads (e.g. NHS-activated sepharose), latex, cellulose, and ferro- or ferrimagnetic particles.

In case that the solid support is a material comprising various entities, e.g. in case that the solid support comprises several beads or particles, it is envisaged within the present invention that, if different compounds are immobilized, on each single entity, e.g. each bead or particle, one or more different compounds are immobilized. Therefore, in case that two compounds are used, it is envisaged within the present invention that on each single entity one or two different compounds are immobilized. If no measures are taken that on one entity only one different compound is immobilized, it is very likely that on each entity all different compounds will be present.

The compound or compounds of the invention may be coupled to the solid support either covalently or non-covalently. Non-covalent binding includes binding via biotin affinity ligands binding to steptavidin matrices.

Preferably, the compound or compounds are covalently coupled to the solid support.

Methods for immobilizing compounds on solid supports are known in the art and further exemplified in the Example section. Especially, the skilled person will understand that if the compound is covalently coupled to said solid support, the residues forming part of said coupling will have to be modified accordingly.

In general, before the coupling, the matrixes can contain active groups such as NHS, Carbodimide etc. to enable the coupling reaction with the immobilization compound. The compound can be coupled to the solid support by direct coupling (e.g. using functional groups such as amino-, sulfhydryl-, carboxyl-, hydroxyl-, aldehyde-, and ketone groups) and by indirect coupling (e.g. via biotin, biotin being covalently attached to the immobilization product of the invention and non-covalent binding of biotin to streptavidin which is bound directly to the solid support).

The linkage to the solid support material may involve cleavable and non-cleavable linkers. The cleavage may be achieved by enzymatic cleavage or treatment with suitable chemical methods.

Therefore, according to a preferred embodiment of the invention, the immobilization product results from a covalent direct or linker mediated attachment of the at least one compound of the invention to the solid support. This linker may be a C₁₋₁₀ alkylene group, which is optionally interrupted or terminated by one or more atoms or functional groups selected from the group consisting of S, O, NH, C(O)O, OC(O), C(O), NHC(O), and C(O)NH and wherein the linker is optionally substituted with one or more substituents independently selected from the group consisting of halogen, OH, NH₂, C(O)H, C(O)NH₂, SO₃H, NO₂, and CN.

When describing the covalent linker to the solid support, the term "C₁₋₁₀ alkylene" means an alkylene chain having 1 - 10 carbon atoms, e.g. methylene, ethylene, n-propylene and the like, wherein each hydrogen of a carbon atom may be replaced by a substituent as indicated herein. The term "C₁₋₆ alkylene" as used herein is defined accordingly.

The term "interrupted" means that the one or more atoms or functional groups are inserted between two carbon atoms of the alkylene chain or -when "terminated"- at the end of said chain.

In a preferred embodiment of the invention, the solid support is selected from the group consisting of agarose, modified agarose, sepharose beads (e.g. NHS-activated sepharose), latex, cellulose, and ferro- or ferrimagnetic particles.

Methods and strategies for choosing appropriate solid supports and for coupling compounds to said solid supports are known in the art (see e.g. Wong, Shan S. Chemistry of protein conjugation and cross-linking (1991), CRC Press, Inc. ISBN 0-8493-5886-8 Chapter 12: Conjugation of proteins to solid matrices, pages 295-318).

In a preferred embodiment, the compound or immobilization product of the invention may further be labeled.

By "labeled" is meant that the respective substance is either directly or indirectly labeled with a molecule which provides a detection signal, e.g. radioisotope, fluorescent tag, chemiluminescent tag, a peptide or specific binding molecules. Specific binding molecules include pairs, such as biotin and streptavidin, digoxin and antidigoxin. The label can directly or indirectly provide a detectable signal. The tag can also be a peptide which can be used, for example, in an enzyme fragment complementation assay (e.g. beta-galactosidase enzyme fragment complementation; Zaman et al., 2006. Assay Drug Dev. Technol. 4(4):411-420). The labeled compounds would be useful not only in imaging techniques but also in assays, both in vitro and in vivo, for identifying histone demethylase interacting compounds by inhibition of binding of the labeled compound, for example in histone demethylase assays that contain such labeled compounds.

Radioisotopes are commonly used in biological applications for the detection of a variety of biomolecules and have proven to be useful in binding assays. Several examples of probes have been designed to incorporate ³H (also written as T for tritium) because it can replace hydrogen in a probe without altering its structure (Fenteany et al., 1995. Science 268:726-731). An "isotopically" or "radio-labeled" compound is a compound of the invention where one or more atoms are replaced or substituted by an atom having an atomic mass or mass number different from the atomic mass or mass number typically found in nature (i.e., naturally occurring). Suitable radionuclides that may be incorporated in compounds of the present invention include but are not limited to ²H (also written D for Deuterium), ¹¹C, ¹³C, ¹⁴C, ¹³N, ¹⁵N, ¹⁵O, ¹⁷O, ¹⁸O, ¹⁸F, ³⁵S, ³⁶Cl, ⁸²Br, ⁷⁵Br, ⁷⁶Br, ⁷⁷Br, ¹²³I, ¹²⁴I, ¹²⁵I and ¹³¹I.

Guidance for the selection and methods for the attachment of fluorescent tags (e.g. fluorescein, rhodamine, dansyl, NBD (nitrobenz-2-oxa-1,3-diazole), BODIPY (dipyrromethene boron difluoride), and cyanine (Cy)-dyes) to small molecule ligands are generally known in the art (Vedvik et al., 2004. Assay Drug Dev. Technol. 2(2): 193-203; Zhang et al., 2005. Analytical Biochemistry 343(1):76-83). The application of fluorescent probes (fluorophores) in assays for high throughput screening (HTS) of kinases was described (Zaman et al., 2003. Comb. Chem. High Throughput Screen 6(4): 313-320). The change of the fluorescent properties after binding of the fluorescent probe to the target histone demethylase can be determined by measuring for example fluorescence polarization (Kashem et al., 2007. J. Biomol. Screening 12(1):70-83), fluorescence resonance energy transfer (FRET; Zhang et al., 2005. Analytical Biochemistry 343(1):76-83) or fluorescence lifetime (Moger et al., 2006. J. Biomol. Screening 11(7): 765-772). In addition, the ALPHAScreen technology can be used where the excitation of a donor bead at 680 nm produces singlet oxygen which can diffuse to an acceptor bead undergoing a chemiluminescent reaction (Glickman et al., 2002. J. Biomol. Screen. 7(1):3-10).

One possible use of the immobilization products of the invention is in the context of the identification of compounds interacting with histone demethylases. Therefore, the present invention also relates to such methods and uses.

In a first aspect of the methods of the invention, the invention therefore relates to a method for the identification of a histone demethylase interacting compound, comprising the steps of
a) providing a protein preparation containing at least one histone demethylase,
b) contacting the protein preparation with the immobilization product of the invention under conditions allowing the formation of one or more different complexes between one of the histone demethylases and the immobilization product,
c) incubating the one or more different complexes with a given compound, and
d) determining whether the compound is able to separate the histone demethylase
   from the immobilization product.

In a second aspect, the present invention relates into a method for the identification of a histone demethylase interacting compound, comprising the steps of
a) providing a protein preparation containing at least one histone demethylase,
b) contacting the protein preparation with the immobilization product of the invention and with a given compound under conditions allowing the formation of one or more different complexes between one of the histone demethylases and the immobilization product, and
c) detecting the complex or the complexes formed in step b).

In a third aspect, the present invention relates to a method for the identification of a histone demethylase interacting compound, comprising the steps of:
a) providing two aliquots of a protein preparation containing at least one histone demethylase,
b) contacting one aliquot with the immobilization product of the invention under conditions allowing the formation of one or more different complexes between one of the histone demethylases and the immobilization product,
c) contacting the other aliquot with the immobilization product of the invention and with a given compound under conditions allowing the formation of one or more different complexes between one of the histone demethylases and the immobilization product, and
d) determining the amount of the complex or the complexes formed in steps b) and c).

In a fourth aspect, the invention relates to a method for the identification of a histone demethylase interacting compound, comprising the steps of:
a) providing two aliquots of a cell preparation comprising each at least one cell containing at least one histone demethylase,
b) incubating one aliquot with a given compound,
c) harvesting the cells of each aliquot,
d) lysing the cells in order to obtain protein preparations,
e) contacting the protein preparations with the immobilization product of the invention under conditions allowing the formation of one or more different complexes between one of the histone demethylases and the immobilization product, and
f) determining the amount of the complex or the complexes formed in each aliquot in step e).

In the context of the present invention, it has been found that the immobilization products of the present invention are suitable for the identification of compounds interacting with histone demethylases.

The immobilization products of the present invention bind several histone demethylases. For example, the following histone demethylases were identified in the biological examples: JHDM1D, JMJD3 and UTY (Table 5 and 9). Consequently, in the context of the present invention, the term "at least one histone demethylase" means that at least one type of histone methylase (e.g. JHDM1D, JMJD3 or UTY) is present in the respective sample, e.g. the cell or the protein preparation.

Furthermore, the skilled person will appreciate that the respective sample will contain one or more histone demethylases of said type. Therefore, the person skilled in the art will understand that in the context of the present invention, with respect to a histone demethylase, the singular and the plural may be used interchangeably.

Preferably the histone demethylase is an enzyme that can demethylate methylated lysine or arginine residues. Even more preferred the histone demethylase is an enzyme that can demethylate methylated lysine residues of histone proteins (Agger et al., 2008. Curr. Opin. Genet. Dev. 18(2):159-168).

Equally preferred, the expression "histone demethylase" denotes a protein that contains a Jumonji C (JmjC) domain. A JmjC domain is known in the art (Agger et al., 2008. Curr. Opin. Genet. Dev. 18(2):159-168).

Consequently, in the methods of the present invention, these immobilization products can be used to identify compounds binding to histone demethylases.

Several histone demethylases have been implicated in human cancers. For example, the GASC1/KDM4C protein which belongs to the JMJD2 family was initially identified as a gene amplified in cell lines from esophageal squamous cell carcinomas. Recent evidence suggests that GASC1 may be involved in a cascade of events that contributes to the maintenance of pluripotency and self renewal of stem cells (Kampranis and Tsichlis, 2009. Adv. Cancer Res. 102:103-169).

According to the present invention, the expression "histone demethylase" relates to both human and other proteins of this family. The expression especially includes functionally active derivatives thereof, or functionally active fragments thereof, or a homologues thereof, or variants encoded by a nucleic acid that hybridizes to the nucleic acid encoding said protein under low stringency conditions. Preferably, these low stringency conditions include hybridization in a buffer comprising 35% formamide, 5X SSC, 50 mM Tris-HCl (pH 7.5), 5 mM EDTA, 0.02% PVP, 0.02% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1-5 hours at 55°C, and washing in a buffer consisting of 2X SSC, 25 mM Tris-HCl (pH 7.4) 5 mM EDTA, and 0.1% SDS for 1.5 hours at 60°C.

Moreover, according to the present invention, the expression "histone demethylase" includes mutant forms said histone demethylases.

In some aspects of the invention, first a protein preparation containing said histone demethylase is provided. The methods of the present invention can be performed with any protein preparation as a starting material, as long as the respective histone demethylase is solubilized in the preparation. Examples include a liquid mixture of several proteins, a cell lysate, a partial cell lysate which contains not all proteins present in the original cell or a combination of several cell lysates. The term "protein preparation" also includes dissolved purified protein.

In another aspect of the invention, aliquots of a cell preparation are provided as the starting material. In the context of the present invention, the term "cell preparation" refers to any preparation containing at least one cell with the desired properties. Suitable cell preparation are described below. The presence of the histone demethylases in a protein preparation of interest can be detected on Western blots probed with antibodies that are specifically directed against said histone demethylase. Alternatively, also mass spectrometry (MS) could be used to detect the histone demethylases (see below).

Cell lysates or partial cell lysates can be obtained by isolating cell organelles (e.g. nucleus, mitochondria, ribosomes, golgi etc.) first and then preparing protein preparations derived from these organelles. Methods for the isolation of cell organelles are known in the art (Chapter 4.2 Purification of Organelles from Mammalian Cells in "Current Protocols in Protein Science", Editors: John.E. Coligan, Ben M. Dunn, Hidde L. Ploegh, David W. Speicher, Paul T. Wingfield; Wiley, ISBN: 0-471-14098-8).

In addition, protein preparations can be prepared by fractionation of cell extracts thereby enriching specific types of proteins such as nuclear proteins (Dignam et al., 1983. Nucleic Acids Res. 11 (5):1475-89).

Furthermore protein preparations from body fluids can be used (e.g. blood, cerebrospinal fluid, peritoneal fluid and urine).

Furthermore, the protein preparation may be a preparation containing the histone demethylase or the histone demethylases which has been recombinantely produced. Methods for the production of recombinant proteins in prokaryotic and eukaryotic cells are widely established (Chapter 5 Production of Recombinant Proteins in "Current Protocols in Protein Science", Editors: John. E. Coligan, Ben M. Dunn, Hidde L. Ploegh, David W. Speicher, Paul T. Wingfield; Wiley, 1995, ISBN: 0-471-14098-8).

In a preferred embodiment of the methods of the invention, the provision of a protein preparation includes the steps of harvesting at least one cell containing the histone demethylase or the histone demethylases and lysing the cell.

Suitable cells for this purpose as well as for the cell preparations used as the starting material in one aspect of the present invention are e.g. those cells or tissues where the histone demethylases are expressed. In any given cell or tissue only a subset of the histone demethylases may be expressed. Therefore it may be necessary to generate multiple protein preparations from a variety of cell types and tissues to cover the histone demethylase family of proteins, especially for selectivity profiling of histone demethylase inhibitors. As established cell lines may not reflect the physiological expression pattern of histone demethylases, primary animal or human cells may be used, for example cells isolated from blood samples.

Therefore, in a preferred embodiment, cells isolated from peripheral blood represent a suitable biological material. Procedures for the preparation and culture of human lymphocytes and lymphocyte subpopulations obtained from peripheral blood (PBLs) are widely known (W.E Biddison, Chapter 2.2 "Preparation and culture of human lymphocytes" in Current Protocols in Cell Biology, 1998, John Wiley & Sons, Inc.). For example, density gradient centrifugation is a method for the separation of lymphocytes from other blood cell populations (e.g. erythrocytes and granulocytes). Human lymphocyte subpopulations can be isolated via their specific cell surface receptors which can be recognized by monoclonal antibodies. The physical separation method involves coupling of these antibody reagents to magnetic beads which allow the enrichment of cells that are bound by these antibodies (positive selection).

As an alternative to primary human cells cultured cell lines (e.g. MOLT-4 cells, Jurkat, Ramos, HL-60 or HeLa cells) can be used.

In a preferred embodiment, the cell is part of a cell culture system and methods for the harvest of a cell out of a cell culture system are known in the art (literature supra).

The choice of the cell will mainly depend on the expression of the histone demethylases, since it has to be ensured that the protein is principally present in the cell of choice. In order to determine whether a given cell is a suitable starting system for the methods of the invention, methods like Westernblot, PCR-based nucleic acids detection methods, Northernblots and DNA-microarray methods ("DNA chips") might be suitable in order to determine whether a given protein of interest is present in the cell.

The choice of the cell may also be influenced by the purpose of the study. If the in vivo efficacy for a given drug needs to be analyzed then cells or tissues may be selected in which the desired therapeutic effect occurs (e.g. T-cells). By contrast, for the elucidation of protein targets mediating unwanted side effects the cell or tissue may be analysed in which the side effect is observed (e.g. cardiomyocytes).

Furthermore, it is envisaged within the present invention that the cell containing the histone demethylases or the histone demethylase may be obtained from an organism, e.g. by biopsy. Corresponding methods are known in the art. For example, a biopsy is a diagnostic procedure used to obtain a small amount of tissue, which can then be examined microscopically or with biochemical methods. Biopsies are important to diagnose, classify and stage a disease, but also to evaluate and monitor drug treatment.

It is encompassed within the present invention that by the harvest of the at least one cell, the lysis is performed simultaneously. However, it is equally preferred that the cell is first harvested and then separately lysed.

Methods for the lysis of cells are known in the art (Karwa and Mitra: Sample preparation for the extraction, isolation, and purification of Nuclei Acids; chapter 8 in "Sample Preparation Techniques in Analytical Chemistry", Wiley 2003, Editor: Somenath Mitra, print ISBN: 0471328456; online ISBN: 0471457817). Lysis of different cell types and tissues can be achieved by homogenizers (e.g. Potter-homogenizer), ultrasonic desintegrators, enzymatic lysis, detergents (e.g. NP-40, Triton X-100, CHAPS, SDS), osmotic shock, repeated freezing and thawing, or a combination of these methods. According to the methods of the invention, the protein preparation containing one or more histone demethylases is contacted with the immobilization product under conditions allowing the formation of a complex between the said histone demethylase and the immobilization product of the invention.

In the present invention, the term "a complex between a histone demethylase and the immobilization product" denotes a complex where the immobilization product interacts with a histone demethylase , e.g. by covalent or, most preferred, by non-covalent binding.

In the context of the present invention, compounds are identified which interfere with the formation of a complex between the immobilization product and a histone demethylase present in a cell or protein preparation. In case that only one histone demethylase is to be detected or present, the formation of one complex is observed and tested. In case that several histone demethylases are to be detected or present, the formation of several, different complexes is observed and tested.

The skilled person will know which conditions can be applied in order to enable the formation of said complex.

In the context of the present invention, the term "under conditions allowing the formation of the complex" includes all conditions under which such formation, preferably such binding is possible. This includes the possibility of having the solid support on an immobilized phase and pouring the lysate onto it. In another preferred embodiment, it is also included that the solid support is in a particulate form and mixed with the cell lysate. Such conditions are known to the person skilled in the art.

In the context of non-covalent binding, the binding between the immobilization product and the histone demethylase is, e.g., via salt bridges, hydrogen bonds, hydrophobic interactions or a combination thereof.

In a preferred embodiment, the steps of the formation of said complex are performed under essentially physiological conditions. The physical state of proteins within cells is described in Petty, 1998 (Howard R. Petty, Chapter 1, Unit 1.5 in: Juan S. Bonifacino, Mary Dasso, Joe B. Harford, Jennifer Lippincott-Schwartz, and Kenneth M. Yamada (eds.) Current Protocols in Cell Biology Copyright © 2003 John Wiley & Sons, Inc. All rights reserved. DOI: 10.1002/0471143030.cb0101s00Online Posting Date: May, 2001Print Publication Date: October, 1998).

The contacting under essentially physiological conditions has the advantage that the interactions between the ligand, the cell preparation (i. e. the histone demethylase to be characterized) and optionally the compound reflect as much as possible the natural conditions. "Essentially physiological conditions" are *inter alia* those conditions which are present in the original, unprocessed sample material. They include the physiological protein concentration, pH, salt concentration, buffer capacity and post-translational modifications of the proteins involved. The term "essentially physiological conditions" does not require conditions identical to those in the original living organism, wherefrom the sample is derived, but essentially cell-like conditions or conditions close to cellular conditions. The person skilled in the art will, of course, realize that certain constraints may arise due to the experimental set-up which will eventually lead to less cell-like conditions. For example, the eventually necessary disruption of cell walls or cell membranes when taking and processing a sample from a living organism may require conditions which are not identical to the physiological conditions found in the organism. Suitable variations of physiological conditions for practicing the methods of the invention will be apparent to those skilled in the art and are encompassed by the term "essentially physiological conditions" as used herein. In summary, it is to be understood that the term "essentially physiological conditions" relates to conditions close to physiological conditions, as e. g. found in natural cells, but does not necessarily require that these conditions are identical.

For example, "essentially physiological conditions" may comprise 50-200 mM NaCl or KCl, pH 6.5-8.5, 20-37°C, and 0.001-10 mM divalent cation (e.g. Mg++, Ca++,); more preferably about 150 m NaCl or KCl, pH7.2 to 7.6, 5 mM divalent cation and often include 0.01-1.0 percent non-specific protein (e.g. BSA). A non-ionic detergent (Tween, NP-40, Triton-X100) can often be present, usually at about 0.001 to 2%, typically 0.05-0.2% (volume/volume). For general guidance, the following buffered aequous conditions may be applicable: 10-250 mM NaCl, 5-50 mM Tris HCl, pH5-8, with optional addition of divalent cation(s) and/or metal chelators and/or non-ionic detergents.

Preferably, "essentially physiological conditions" mean a pH of from 6.5 to 7.5, preferably from 7.0 to 7.5, and / or a buffer concentration of from 10 to 50 mM, preferably from 25 to 50 mM, and / or a concentration of monovalent salts (e.g. Na or K) of from 120 to 170 mM, preferably 150 mM. Divalent salts (e.g. Mg or Ca) may further be present at a concentration of from 1 to 5 mM, preferably 1 to 2 mM, wherein more preferably the buffer is selected from the group consisting of Tris-HCl or HEPES.

The skilled person will appreciate that between the individual steps of the methods of the invention, washing steps may be necessary. Such washing is part of the knowledge of the person skilled in the art. The washing serves to remove non-bound components of the cell lysate from the solid support. Nonspecific (e.g. simple ionic) binding interactions can be minimized by adding low levels of detergent or by moderate adjustments to salt concentrations in the wash buffer.

According to the identification methods of the invention, the read-out system is either the detection or determination of a histone demethylase (first aspect of the invention), the detection of the complex between a histone demethylase and the immobilization product (second aspect of the invention), or the determination of the amount of the complex between a histone demethylase and the immobilization product (second, third and fourth aspect of the invention).

In the method according to the first aspect of the invention, the detection or determination of the amount of separated histone demethylase is preferably indicative for the fact that the compound is able to separate the histone demethylase from the immobilization product. This capacity indicates that the respective compound interacts, preferably binds to the histone demethylase, which is indicative for its therapeutic potential.

In one embodiment of the method according to the second aspect of the invention, the complex formed during the method of the invention is detected. The fact that such complex is formed preferably indicates that the compound does not completely inhibit the formation of the complex. On the other hand, if no complex is formed, the compound is presumably a strong interactor with the histone demethylase, which is indicative for its therapeutic potential.

According to a preferred embodiment of the second aspect of the invention, the complex between one histone demethylase and the immobilization product is detected by determining its amount.

According to the methods of the second, third and fourth aspect of the invention the amount of the complex formed during the method is determined. In general, the less complex in the presence of the respective compound is formed, the stronger the respective compound interacts with the histone demethylase, which is indicative for its therapeutic potential.

The detection of the complex formed according to the second aspect of the invention can be performed by using labeled antibodies directed against the histone demethylase and a suitable readout system.

In the course of the second, third and fourth aspect of the invention, it is preferred that the histone demethylase is separated from the immobilization product in order to determine the amount of said complex.

According to invention, separating means every action which destroys the interactions between the immobilization compound and the histone demethylase. This includes in a preferred embodiment the elution of the histone demethylase from the immobilization compound.

The elution can be achieved by using non-specific reagents as described in detail below (ionic strength, pH value, detergents). In addition, it can be tested whether a compound of interest can specifically elute the histone demethylase from the immobilization compound. Such histone demethylase interacting compounds are described further in the following sections.

Such non-specific methods for destroying the interaction are principally known in the art and depend on the nature of the ligand enzyme interaction. Principally, change of ionic strength, the pH value, the temperature or incubation with detergents are suitable methods to dissociate the target enzymes from the immobilized compound. The application of an elution buffer can dissociate binding partners by extremes of pH value (high or low pH; e.g. lowering pH by using 0.1 M citrate, pH2-3), change of ionic strength (e.g. high salt concentration using NaI, KI, MgCl₂, or KCl), polarity reducing agents which disrupt hydrophobic interactions (e.g. dioxane or ethylene glycol), or denaturing agents (chaotropic salts or detergents such as Sodium-docedyl-sulfate, SDS; Review: Subramanian A., 2002, Immunoaffinty chromatography).

In some cases, the solid support has preferably to be separated from the released material. The individual methods for this depend on the nature of the solid support and are known in the art. If the support material is contained within a column the released material can be collected as column flowthrough. In case the support material is mixed with the lysate components (so called batch procedure) an additional separation step such as gentle centrifugation may be necessary and the released material is collected as supernatant. Alternatively magnetic beads can be used as solid support so that the beads can be eliminated from the sample by using a magnetic device.

In step d) of the method according to the first aspect of the invention, it is determined if the histone demethylase has been separated from the immobilization product of the invention. This may include the detection of the histone demethylase or the determination of the amount of the histone demethylase.

Consequently, at least in preferred embodiments of all identification methods of the invention, methods for the detection of a separated histone demethylase or for the determination of their amount are used. Such methods are known in the art and include physico-chemical methods such as protein sequencing (e.g. Edmann degradation), analysis by mass spectrometry methods or immunodetection methods employing antibodies directed against the histone demethylase.

Throughout the invention, if an antibody is used in order to detect a histone demethylase or in order to determine its amount (e.g. via ELISA), the skilled person will understand that, if a specific histone demethylase is to be detected or if the amount of a histone demethylase is to be determined, a specific antibody may be used (Gray et al., 2006. J. Biol. Chem. 280(31):28507-28518). As indicated above, such antibodies are known in the art. Furthermore, the skilled person is aware of methods for producing the same.

Preferably, a histone demethylase is detected or the amount of a histone demethylase is determined by mass spectrometry or immunodetection methods.

The identification of proteins with mass spectrometric analysis (mass spectrometry, MS) is known in the art (Shevchenko et al., 1996, Analytical Chemistry 68: 850-858; Mann et al., 2001. Annual Review of Biochemistry 70, 437-473) and is further illustrated in the example section.

Preferably, the mass spectrometry analysis is performed in a quantitative manner, for example by stable isotope labeling to create a specific mass tag that can be recognized by a mass spectrometer and at the same time provide the basis for quantification. These mass tags can be introduced into proteins or peptides metabolically, by chemical means, enzymatically, or provided by spiked synthetic peptide standards (Bantscheff et al., 2007; Anal. Bioanal. Chem. 389(4):1017-1031).

Preferably, the stable isotope is introduced into proteins by metabolic labeling during cell growth and division, for example by the stable isotope labeling by amino acids in cell culture (SILAC) approach (Ong et al., 2002; Mol. Cell. Proteomics. 1 (5):376-386).

Preferably, the mass spectrometry analysis is performed in a quantitative manner, for example by using iTRAQ technology (isobaric tags for relative and absolute quatification) or cICAT (cleavable isotope-coded affinity tags) (Wu et al., 2006. J. Proteome Res. 5, 651-658).

According to a further preferred embodiment of the present invention, the characterization by mass spectrometry (MS) is performed by the identification of proteotypic peptides of the histone demethylase. The idea is that the histone demethylase is digested with proteases and the resulting peptides are determined by MS. As a result, peptide frequencies for peptides from the same source protein differ by a great degree, the most frequently observed peptides that "typically" contribute to the identification of this protein being termed "proteotypic peptide". Therefore, a proteotypic peptide as used in the present invention is an experimentally well observable peptide that uniquely identifies a specific protein or protein isoform.

According to a preferred embodiment, the characterization is performed by comparing the proteotypic peptides obtained in the course of practicing the methods of the invention with known proteotypic peptides. Since, when using fragments prepared by protease digestion for the identification of a protein in MS, usually the same proteotypic peptides are observed for a given histone demethylase, it is possible to compare the proteotypic peptides obtained for a given sample with the proteotypic peptides already known for histone demethylases and thereby identifying the histone demethylase being present in the sample.

As an alternative to mass spectrometry analysis, the eluted histone demethylase (including coeluted binding partners such as regulatory subunits), can be detected or its amount can be determined by using a specific antibody directed against the histone demethylase.

Furthermore, in another preferred embodiment, once the identity of the coeluted binding partner (e.g. regulatory subunit) has been established by mass spectrometry analysis, each binding partner can be detected with specific antibodies directed against this protein.

Suitable antibody-based assays include but are not limited to Western blots, ELISA assays, sandwich ELISA assays and antibody arrays or a combination thereof. The establishment of such assays is known in the art (Chapter 11, Immunology, pages 11-1 to 11-30 in: Short Protocols in Molecular Biology. Fourth Edition, Edited by F.M. Ausubel et al., Wiley, New York, 1999).

These assays can not only be configured in a way to detect and quantify a histone demethylase interacting protein of interest, for example a component of a histone demethylase protein complex (Gray et al., 2006. J. Biol. Chem. 280(31):28507-28518), but also to analyse posttranslational modification patterns such as phosphorylation or ubiquitin modification.

Furthermore, the identification methods of the invention involve the use of compounds which are tested for their ability to be a histone demethylase interacting compound. Principally, according to the present invention, such a compound can be every molecule which is able to interact with the histone demethylase, eg. by inhibiting its binding to the immobilization product of the invention. Preferably, the compound has an effect on the histone demethylase, e.g. a stimulatory or inhibitory effect.

Preferably, said compound is selected from the group consisting of synthetic or naturally occurring chemical compounds or organic synthetic drugs, more preferably small molecule organic drugs or natural small molecule compounds. Preferably, said compound is identified starting from a library containing such compounds. Then, in the course of the present invention, such a library is screened.

Such small molecules are preferably not proteins or nucleic acids. Preferably, small molecules exhibit a molecular weight of less than 1000 Da, more preferred less than 750 Da, most preferred less than 500 Da.

A "library" according to the present invention relates to a (mostly large) collection of (numerous) different chemical entities that are provided in a sorted manner that enables both a fast functional analysis (screening) of the different individual entities, and at the same time provide for a rapid identification of the individual entities that form the library. Examples are collections of tubes or wells or spots on surfaces that contain chemical compounds that can be added into reactions with one or more defined potentially interacting partners in a high-throughput fashion. After the identification of a desired "positive" interaction of both partners, the respective compound can be rapidly identified due to the library construction. Libraries of synthetic and natural origins can either be purchased or designed by the skilled artisan.

Examples of the construction of libraries are provided in, for example, Breinbauer R, Manger M, Scheck M, Waldmann H. Natural product guided compound library development. Curr. Med. Chem. 2002; 9(23):2129-2145, wherein natural products are described that are biologically validated starting points for the design of combinatorial libraries, as they have a proven record of biological relevance. This special role of natural products in medicinal chemistry and chemical biology can be interpreted in the light of new insights about the domain architecture of proteins gained by structural biology and bioinformatics. In order to fulfill the specific requirements of the individual binding pocket within a domain family it may be necessary to optimise the natural product structure by chemical variation. Solid-phase chemistry is said to become an efficient tool for this optimisation process, and recent advances in this field are highlighted in this review article. The current drug discovery processes in many pharmaceutical companies require large and growing collections of high quality lead structures for use in high throughput screening assays. Collections of small molecules with diverse structures and "drug-like" properties have, in the past, been acquired by several means: by archive of previous internal lead optimisation efforts, by purchase from compound vendors, and by union of separate collections following company mergers. Although high throughput/combinatorial chemistry is described as being an important component in the process of new lead generation, the selection of library designs for synthesis and the subsequent design of library members has evolved to a new level of challenge and importance. The potential benefits of screening multiple small molecule compound library designs against multiple biological targets offers substantial opportunity to discover new lead structures.

In a preferred embodiment of the second and third aspect of the invention, the histone demethylase containing protein preparation is first incubated with the compound and then with the immobilization product. However, the simultaneous incubation of the compound and the immobilization product of the invention (coincubation) with the histone demethylase containing protein preparation is equally preferred (competitive binding assay).

In case that the incubation with the compound is first, the histone demethylase is preferably first incubated with the compound for 10 to 60 minutes, more preferred for 30 to 45 minutes at a temperature of 4°C to 37°C, more preferred at 4°C to 25°C, most preferred at 4°C. Preferably compounds are used at concentrations ranging from 1 nM to 1 mM, preferably from 1 nM to 100 µM, preferably from 1 nM to 10 µM. The second step, contacting with the immobilized ligand, is preferably performed for 10 to 60 minutes at 4°C.

In case of simultaneous incubation, the histone demethylase is preferably simultaneously incubated with the compound and the immobilization product of the invention for 30 to 120 minutes, more preferred for 60 to 120 minutes at a temperature of 4°C to 37°C, more preferred at 4°C to 25°C, most preferred at 4°C. Preferably compounds are used at concentrations ranging from 1 nM to 1 mM, preferably from 1 nM to 100 µM, preferably from 1 nM to 10 µM.

Furthermore, steps a) to c) of the second aspect of the invention may be performed with several protein preparations in order to test different compounds. This embodiment is especially interesting in the context of medium or high throughput screenings.

In a preferred embodiment of the method of the invention according to the third or fourth aspect, the amount of the complex formed in step c) is compared to the amount formed in step b)

In a preferred embodiment of the method of the invention according to the third or fourth aspect, a reduced amount of the complex formed in step c) in comparison to step b) indicates that a histone demethylase is a target of the compound. This results from the fact that in step c) of this method of the invention, the compound competes with the immobilized compound for the binding of the histone demethylase. If less histone demethylase is present in the aliquot incubated with the compound, this means preferably that the compound has competed with the inhibitor for the interaction with the enzyme and is, therefore, a direct target of the protein and vice versa.

Preferably, the identification methods of the invention are performed as a medium or high throughput screening.

The interaction compound identified according to the present invention may be further characterized by determining whether it has an effect on the histone demethylase, for example on its histone demethylase activity (Hamada et al, 2010. J. Med. Chem. 53(15):5629-5638).

The compounds identified according to the present invention may further be optimized in terms of potency and selectivity. An example for lead optimization of histone demethylase inhibitors was reported (Hamada et al, 2010. J. Med. Chem. 53(15):5629-5638).

The invention further relates to a method for the preparation of a pharmaceutical composition comprising the steps of
a) identifying a histone demethylase interacting compound as described above, and
b) formulating the interacting compound to a pharmaceutical composition.

Methods for the formulation of identified compounds are known in the art. Furthermore, it is known in the art how to administer such pharmaceutical compositions.

The obtained pharmaceutical composition can be used for the prevention or treatment of diseases where the respective histone demethylase plays a role, e.g. for the prevention or treatment of cancer (Kampranis and Tsichlis, 2009. Adv. Cancer Res. 102:103-169). For example, histone demethylase inhibitors may be useful for the treatment of inflammatory diseases, cancer or metabolic diseases.

The invention further relates to a method for the purification of a histone demethylase, comprising the steps of
a) providing a protein preparation containing said histone demethylase,
b) contacting the protein preparation with the immobilization product of the invention under conditions allowing the formation of a complex between the histone demethylase and the immobilization product, and
c) separating the histone demethylase from the immobilization product.

As mentioned above, it has been surprisingly found that the compound of the invention and therefore also the immobilization product of the invention is a ligand which recognizes the histone demethylases mentioned above. This enables efficient purification methods for said histone demethylases.

With respect to the histone demethylases, the protein preparation containing the histone demethylases, the conditions for contacting with the immobilization product of the invention, the immobilization product of the invention, the complex between the histone demethylases and the immobilization product of the invention, the separation of the histone demethylases from the immobilization product of the invention, and the detection of the histone demethylases or the determination of its amount, the embodiments as defined above for the identification methods of the invention also apply to the purification method of the invention.

In a preferred embodiment, the purification method of the invention further comprises after step c) the identification of proteins being capable of binding to said histone demethylases. This is especially interesting when the formation of the complex is performed under essentially physiological conditions, because it is then possible to preserve the natural condition of the enzyme which includes the existence of binding partners, enzyme subunits or post-translational modifications, which can then be identified with the help of mass spectrometry.

Consequently, in a preferred embodiment, the purification method of the invention further comprises after step c) the determination whether the histone demethylase is further posttranslationally modified, e.g. by ubiquitin modification.

The binding proteins or the posttranslational modifications can be determined as explained above for the detection of histone demethylases or the determination of the amount of histone demethylases. Preferably, said methods include mass spectrometry of immunodetection methods as described above.

The invention further relates to a method for determining the presence of one or more histone demethylases in a sample, comprising the steps of:
a) providing a protein preparation expected to contain said one or more histone demethylases,
b) contacting the protein preparation with the immobilization product of the invention under conditions allowing the formation of a complex between one of the histone demethylases and the immobilization product, and
c) detecting whether one or more histone demethylases have formed a complex with the immobilization product.

In a preferred embodiment of the invention, said detecting in step c) is performed by separating said one or more histone demethylases from the immobilization product and further identification of said one or more histone demethylases.

Said identification may be performed by mass spectrometry or immunodetection methods as described above.

According to an especially preferred embodiment of this method of the invention, the histone demethylase contains at least one mutation.

With respect to said one or more histone demethylases, the protein preparation containing said histone demethylases, the conditions for contacting with the immobilization product of the invention, the immobilization product of the invention, the complex between said histone demethylase and the immobilization product of the invention, the separation of histone demethylases from the immobilization product of the invention, and the detection of histone demethylases or the determination of its amount, the embodiments as defined above for the identification methods of the invention also apply to the purification method of the invention.

The invention further relates to the use of the immobilization product of the invention for the identification of a histone demethylase interacting compound and for the purification of a histone demethylase. The embodiments as defined above also apply to the uses of the invention.

The invention further relates to a kit comprising the immobilization product of the invention. Such a kit is especially useful for performing the methods of the invention. Further components of the kit may be antibodies for the detection of histone demethylase proteins. Such antibodies and their use are known in the art and they are commercially available (Gray et al., 2006. J. Biol. Chem. 280(31):28507-28518). Furthermore, the kit may contain further auxiliary components like buffers, means for the detection of antibodies, and positive controls. Such components are known in the art. The invention further relates to a method of diagnosing an inflammatory disease, a proliferative disease or a metabolic disease, wherein an immobilization product of the invention is used for determining the presence of a histone demethylase in the diseased cell or tissue.

In a further preferred embodiment of the methods or uses of the present invention, the affinity of the histone demethylase interacting compound for the histone demethylase is determined. This can be done by incubating different aliquots of the protein preparation or cell preparation with different amounts of the compound and subsequently correlating the amount of complexes with the concentration of the compound. Plotting the amount of complexes against the concentration of the compounds will in most cases result in a curve with sigmoidal shape, with which the IC₅₀ value or the K_{D} value of the compound for the histone demethylase can be determined according to standard methods and as described e.g. in Bantscheff et al, Nature Biotechnology 25:1035-1044 (2007).

The invention is further illustrated by the following figures and examples, which are not considered as being limiting for the scope of protection conferred by the claims of the present application. In case where in the following examples the term "affinity matrix" is used, this term refers to an immobilization product as defined in the present application.

### Brief description of the figures

**Figure 1****:** Amino acid sequence of human JHDM1D (IPI00418567.4). Peptides identified by mass spectrometry are underlined.
**Figure 2****:** Amino acid sequence of human JMJD3 (IPI00550090.7). Peptides identified by mass spectrometry are underlined.

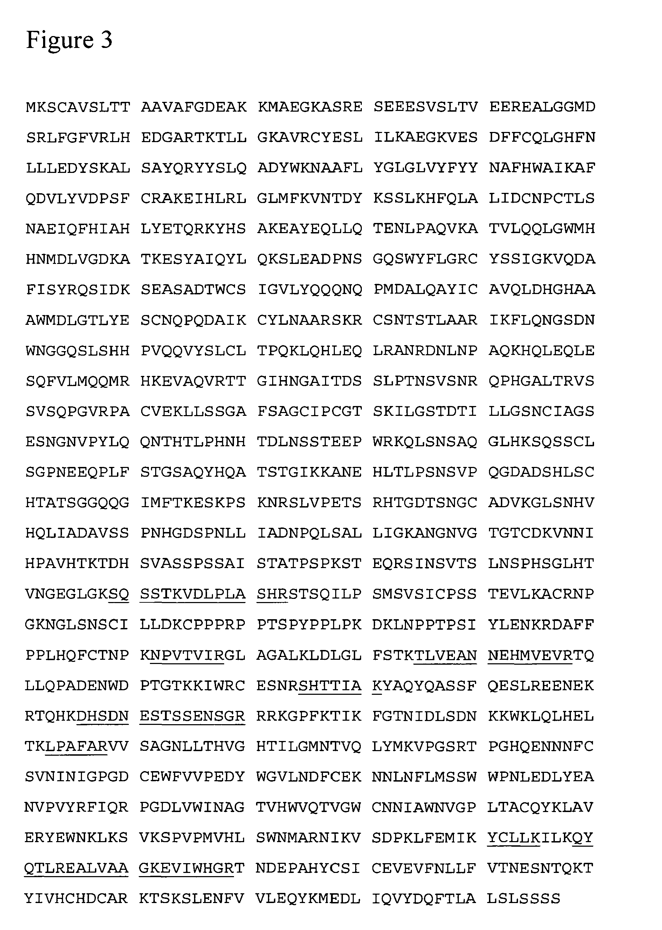

**Figure 3****:** Amino acid sequence of human UTY (IPI00023013.3). Peptides identified by mass spectrometry are underlined.

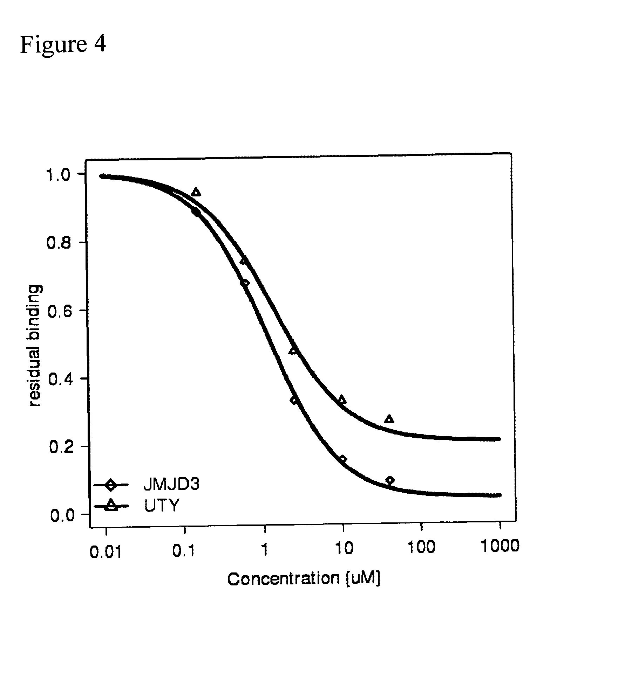

**Figure 4****:** Concentration response curves for reference example 2 binding to Flag-JMJD3(1142-1682) (IC₅₀ = 1.2 µM) and endogenous UTY ) (IC₅₀ = 1.4 µM). The residual binding is plotted against the compound concentration (µM).

### Examples

### Synthesis of compounds

The following examples describe the synthesis of compounds and methods for their immobilization on a solid support yielding the affinity matrix used in the biological examples for the capturing of histone demethylases from cell lysates.

**Table 1: Abbreviations**

| | |
|---|---|
| aq. | Aqueous |
| cv | Column Volumes |
| DCM | Dichloromethane |
| DIPEA | Diisopropylethylamine |
| DMAP | 4-dimethylaminopyridine |
| DMF | N,N-dimethylformamide |
| DMSO | Dimethyl sulfoxide |
| Et₂O | Diethyl ether |
| EtOAc | Ethyl acetate |
| EtOH | Ethanol |
| g | grams |
| h | hours |
| HCl | Hydrochloric acid |
| HPLC | High performance Liquid Chromatography |
| LCMS | Liquid chromatography mass spectroscopy |
| M | Molar |
| MDAP | Mass-directed autopreparative High performance Layer Chromatography |
| MeCN | Acetonitrile |
| MeOH | Methanol |
| mg | Milligrams |
| min | Minutes |
| mL | Millilitres |
| µL | Microlitres |
| µM | Micromolar |
| mmol | Millimoles |
| mol% | Molar percent |
| NMR | Nuclear magnetic resonance |
| Pd(PPh₃)₄ | Tetrakis(triphenylphosphine)palladium(0) |
| rt | Room temperature |
| Rt | Retention time |
| s | seconds |
| sat. | saturated |
| Si | Silica |
| tert | Tertiary |
| T3P | Propane phosphonic acid anhydride |
| TFA | Trifluoroacetic acid |
| THF | Tetrahydrofuran |

### Analytical Methods

LCMS (Method A) was conducted on an Acquity UPLC BEH C18 column (50mm x 2.1mm i.d. 1.7µm packing diameter) at 40 degrees centigrade, eluting with 0.1% v/v solution of Formic Acid in Water (Solvent A) and 0.1 % v/v solution of Formic Acid in Acetonitrile (Solvent B) using the following elution gradient 0-1.5min 3 - 100% B, 1.5-1.9min 100% B, 1.9 - 2.1min 3% B at a flow rate of 1ml/min. The UV detection was a summed signal from wavelength of 210nm to 350nm. The mass spectra were recorded on a Waters ZQ Mass Spectrometer using Alternate-scan Positive and Negative Electrospray. Ionisation data was rounded to the nearest integer.

LCMS (Method B) was conducted on an Acquity UPLC BEH C18 column (50mm x 2.1mm i.d. 1.7µm packing diameter) at 40 degrees centigrade, eluting with 10 mM Ammonium Bicarbonate in water adjusted to pH 10 with Ammonia solution (Solvent A) and Acetonitrile (Solvent B) using the following elution gradient 0-1.5min 1 - 97% B, 1.5-1.9min 97% B, 1.9 - 2.1min 100% B at a flow rate of 1ml/min. The UV detection was a summed signal from wavelength of 210nm to 350nm. The mass spectra were recorded on a Waters ZQ Mass Spectrometer using Alternate-scan Positive and Negative Electrospray. Ionisation data was rounded to the nearest integer.

LCMS (Method C) was conducted on an Acquity UPLC BEH C18 column (50mm x 2.1mm i.d. 1.7µm packing diameter) at 40 degrees centigrade, eluting with 0.1% v/v solution of Trifluoroacetic Acid in Water (Solvent A) and 0.1% v/v solution of Trifluoroacetic Acid in Acetonitrile (Solvent B) using the following elution gradient 0-1.5min 3 - 100% B, 1.5-1.9min 100% B, 1.9 - 2.0min 3% B at a flow rate of 1ml/min. The UV detection was a summed signal from wavelength of 210nm to 350nm. The mass spectra were recorded on a Waters ZQ Mass Spectrometer using Alternate-scan Positive and Negative Electrospray. Ionisation data was rounded to the nearest integer.

LCMS (Method D) was conducted on a HALO C18 Column (50mm x 4.6 mm i.d. 2.7 µm packing diameter) at 40 degrees centigrade, eluting with Water (Solvent A) and Acetonitrile (Solvent B) using the following elution gradient 0-1.0min 5 - 95% B, 1.0-2.0min - 95%, 2.0-2.01min - 95-5%, 2.01-2.5min - 5% at a flow rate of 1.8ml/min. The UV detection was a summed signal from wavelength of 214nm to 254nm.

¹H NMR spectra were recorded using a Varian NMR 400 MHz, referenced to tetramethylsilane.

Silica chromatography techniques include either automated (Flashmaster, Biotage SP4) techniques or manual chromatography on pre-packed cartridges (SPE) or manually-packed flash columns.

Where compounds have been purified by mass-directed autopreparative chromatography (MDAP), one of the following methods may be used:

MDAP (Method E). The HPLC analysis was conducted on an XBridge C18 column (100mm x 30mm i.d. 5µm packing diameter) at ambient temperature, eluting with 10mM Ammonium Bicarbonate in water adjusted to pH 10 with Ammonia solution (Solvent A) and Acetonitrile (Solvent B) using an elution gradient of between 0 and 100% over 15 or 25 minutes. The UV detection was an averaged signal from wavelength of 210nm to 350nm. The mass spectra were recorded on a Waters ZQ Mass Spectrometer using Alternate-scan Positive and Negative Electrospray. Ionisation data was rounded to the nearest integer.

MDAP (Method F) The HPLC was conducted on a Gemini C18 column (150mm x 21.2mm i.d. 5µm packing diameter), eluting with Acetonitrile (Solvent A) and a 0.1% solution of Trifluoroacetic acid in water (Solvent B) using the following elution gradient 0-10min 40-50%B, 10-11min 50-5%B, 11-14min 5%B, 14-15min 5 -40%B at a flow rate of 20ml/min. The UV detection was a summed signal from wavelength of 214nm to 254 nm.

When the name of a commercial supplier is given after the name of a compound or a reagent, for instance "compound X (Aldrich)" or "compound X / Aldrich", this means that compound X is obtainable from a commercial supplier, such as the commercial supplier named.

Similarly, when a literature or a patent reference is given after the name of a compound, for instance compound Y (EP 0 123 456), this means that the preparation of the compound is described in the named reference.

The names of the above mentioned Examples have been obtained using the compound naming programme "ACD Name Pro 6.02".

### Intermediate 1

### 4,6-dichloro-2-(2-pyridinyl)pyrimidine

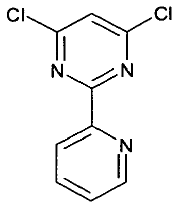

The preparation of 4,6-dichloro-2-(2-pyridinyl)pyrimidine is known in the literature (Ref: WO 2009/087224, CAS No.: 10235-65-1).

### Intermediate 2

### Ethyl N-[6-chloro-2-(2-pyridinyl)-4-pyrimidinyl]-β-alaninate

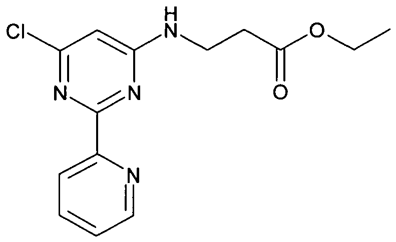

Hunig's base (11.94 mL, 68.3 mmol) was added over 1 min to a stirred suspension of 4,6-dichloro-2-(pyridin-2-yl)pyrimidine (5.15 g, 22.78 mmol) and ethyl 3-aminopropanoate hydrochloride (3.85 g, 25.06 mmol) in 1,4-Dioxane (55 mL) at rt under N₂. The resultant suspension was heated at 60 °C for 6 h by which time a solution had formed and then allowed to stand at rt for 72 h. The solution was then reheated to 60 °C for 3 h. Upon cooling, the solvent was evaporated under reduced pressure to give an orange oil. The oil was dissolved in CH₂Cl₂ (400 mL) and then washed with sat. aq. NaHCO₃ (2 x 100 mL), brine (100 mL) and then passed through a hydrophobic frit. The solution was evaporated under reduced pressure to give an orange oil which was then dried under high vaccum at 40 °C for 12 h to give the title compound as an orange solid (6.94 g, 99%).

LCMS (Method A): Rt = 0.70min, MH⁺ 307

### Intermediate 3

### 4-[2-(1,3-dioxolan-2-yl)ethyl]-2-pyridinecarbonitrile

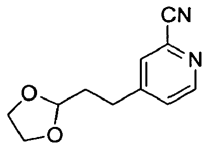

To a dry microwave vial under nitrogen was added bromo[2-(1,3-dioxolan-2-yl)ethyl]zinc (5774 µL, 2.89 mmol, 0.5M solution in THF)/(Aldrich) and 4-chloro-2-pyridinecarbonitrile (250 mg, 1.804 mmol)/(Aldrich). N₂ was bubbled through the mixture for 5 min. Pd(PPh₃)₄ (104 mg, 0.090 mmol) was added and N₂ bubbled through the brown suspension for a further 2 min. The reaction mixture was then heated in a microwave to 110 °C and stirred for 1 h. The reaction mixture was cooled and quenched by the addition of NH₄Cl solution (20 mL) and water (20 mL). The organics were extracted with Et₂O (30 mL) and the layers separated. The aqueous layer was further extracted with Et₂O (2 x 20 mL) and the combined organics dried (Na₂SO₄) and concentrated *in vacuo* to afford the crude product as a yellow oil. The crude product was further purified by silica chromatography, eluting with 0% to 100% ethyl acetate/cyclohexane to give the title compound (151 mg, 41 % yield).

LCMS (Method B): Rt = 0.75 min, MH+ = 205.1.

### Intermediate 4

### 2-{4-[2-(1,3-dioxolan-2-yl)ethyl]-2-pyridinyl}-6-hydroxy-4(1H)-pyrimidinone

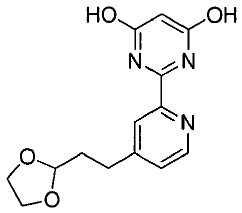

To a solution of 4-[2-(1,3-dioxolan-2-yl)ethyl]-2-pyridinecarbonitrile (1.22 g, 5.99 mmol) in Methanol (11.6 mL) was added sodium methoxide (0.259 g, 1.197 mmol, 25-30% w/w in MeOH) and the reaction stirred at rt for ~1.5 h. Ammonium chloride (0.480 g, 8.98 mmol) was added and the reaction mixture stirred at rt. Diethyl propanedioate (4.56 ml, 29.9 mmol) and sodium methoxide (10.35 g, 47.9 mmol) were added and the reaction heated to reflux (80 °C) for 8.5 h. Further sodium methoxide (10.35 g, 47.9 mmol) and diethyl propanedioate (4.56 ml, 29.9 mmol) were added and heating continued for a further 16 h. The reaction was then allowed to cool and concentrated *in vacuo.* EtOH (50 mL) was added and the reaction mixture sonicated for 5 min. The resultant suspension was filtered and the residue washed with further EtOH (3 x 40 mL). The combined filtrate was concentrated *in vacuo* to afford the title compound as an orange solid (8.31 g). Material was used without further purification in the subsequent reaction.

LCMS (Method A): Rt = 0.59 min, MH+ = 290.1.

### Intermediate 5

### 2-{4-[2-(1,3-dioxolan-2-yl)ethyl]-2-pyridinyl}-4,6-pyrimidinediyl bis(4-methylbenzenesulfonate)

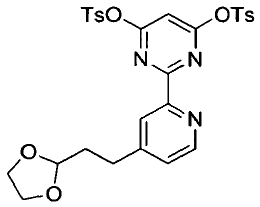

To a solution of 2-{4-[2-(1,3-dioxolan-2-yl)ethyl]-2-pyridinyl}-6-hydroxy-4(1*H*)-pyrimidinone (8.31 g, 5.75 mmol, 20% purity) in DCM (40 mL) was added sequentially triethylamine (5.29 mL, 37.9 mmol), 4-methylbenzenesulfonyl chloride (5.26 g, 27.6 mmol) and DMAP (0.140 g, 1.149 mmol) and the resultant solution stirred at rt for 16 h. Further triethylamine (5.29 mL, 37.9 mmol) and 4-methylbenzenesulfonyl chloride (5.26 g, 27.6 mmol) were added and the reaction stirred for a further 2 h. The reaction mixture was then quenched by the addition of water (50 mL), further DCM (30 mL) was added and the layers separated. The aqueous phase was further extracted with DCM (2 x 40 mL) and the combined organic phase was dried over Na₂SO₄, filtered and concentrated *in vacuo* to give a brown oil. The crude product was purified by column chromatography using a gradient of 10 to 100% EtOAc/cyclohexane to give the title product as a pale cream foam (2.35 g, 68% yield).

LCMS (Method A): Rt = 1.33 min, MH+ = 598.4.

### Intermediate 6

### Ethyl N-(2-{4-[2-(1,3-dioxolan-2-yl)ethyl]-2-pyridinyl}-6-{[(4-methylphenyl)sulfonyl]oxy}-4-pyrimidinyl)-β-alaninate

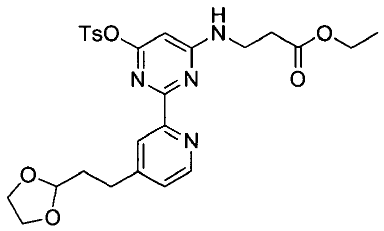

2-{4-[2-(1,3-dioxolan-2-yl)ethyl]-2-pyridinyl}-4,6-pyrimidinediyl bis(4-methylbenzenesulfonate) (3 x 1.0 g, 1.67 mmol and 1 x 857 mg, 1.43 mmol) were dissolved in MeCN (3 x 15 mL and 1 x 12 mL, respectively) in 4 microwave vials equipped with stirrers. To each was added ethyl β-alaninate hydrochloride salt (3 x 265 mg, 1.72 mmol and 1 x 227 mg, 1.48 mmol, respectively) followed by triethylamine (3 x 688 µL, 4.94 mmol and 1 x 590 µL, 4.23 mmol, respectively). The vials were heated at 100 °C for 20 min using a microwave. The mixtures were combined together and concentrated under reduced pressure to give an orange viscous liquid. The liquid was purified by silica chromatography, eluting with 0 to 50% of a 10% solution of MeOH in EtOAc/EtOAc to give the title compound as a colourless waxy oil, 1.4 g (40%).

LCMS (Method A): Rt = 0.93 min, MH+ = 543.3.

### Intermediate 7

### Ethyl N-[2-{4-[2-(1,3-dioxolan-2-yl)ethyl]-2-pyridinyl}-6-(1,2,4,5-tetrahydro-3H-3-benzazepin-3-yl)-4-pyrimidinyl]-β-alaninate

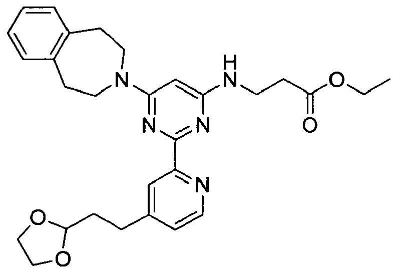

To a solution of ethyl *N*-(2-{4-[2-(1,3-dioxolan-2-yl)ethyl]-2-pyridinyl}-6-{[(4-methylphenyl)sulfonyl]oxy}-4-pyrimidinyl)-β-alaninate (385 mg, 0.710 mmol) in isopropanol (12 mL) in a microwave vial was added 2,3,4,5-tetrahydro-1*H-*3-benzazepine (522 mg, 3.55 mmol). The resultant solution was heated to 150 °C for 1 h. The reaction mixture was then diluted with EtOAc (10 mL) and concentrated *in vacuo* to give the crude product as a yellow oil. The crude product was purified by column chromatography using a gradient of 0% to 40% of a 20% solution of MeOH in DCM/DCM to give the title product as a yellow oil, 219 mg (60%).

LCMS (Method B): Rt = 1.23 min, MH+ = 518.3.

### Intermediate 8

### Ethyl N-[2-[4-(3-oxopropyl)-2-pyridinyl]-6-(1,2,4,5-tetrahydro-3H-3-benzazepin-3-yl)-4-pyrimidinyl]-β-alaninate

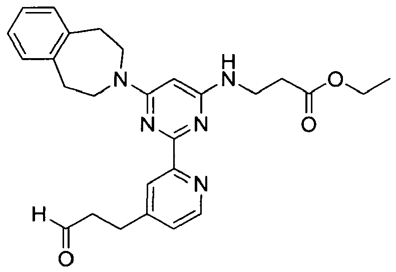

A solution of ethyl *N*-[2-{4-[2-(1,3-dioxolan-2-yl)ethyl]-2-pyridinyl}-6-(1,2,4,5-tetrahydro-3*H*-3-benzazepin-3-yl)-4-pyrimidinyl]-β-alaninate (51 mg, 0.099 mmol) in Acetic Acid (1.5 mL) / Water (0.375 mL) was heated to 80 °C and stirred for 16 h. The reaction mixture was diluted with DCM (20 mL) and then quenched by the addition of NaHCO₃ solution. The quenched reaction was stirred until the effervescence ceased and the layers then separated. The aqueous layer was extracted with further DCM (2 x 20 mL) and the combined organics washed sequentially with NaHCO₃ solution (20 mL) and then brine (20 mL). The organics were dried over Na₂SO₄ then concentrated *in vacuo.* The crude product was re-dissolved in DCM (2 mL) and a small aliquot (~0.1 mL) removed and dried under a stream of N₂ to allow an NMR to be taken. The remainder of the solution was concentrated to afford the crude title compound as a yellow-green oil, 43 mg, (92%) which was used directly in the subsequent reactions.

LCMS (Method B): Rt = 1.18 min, MH+ = 474.2.

### Intermediate 9

### Ethyl N-[2-{4-[3-(methylamino)propyl]-2-pyridinyl}-6-(1,2,4,5-tetrahydro-3H-3-benzazepin-3-yl)-4-pyrimidinyl]-β-alaninate

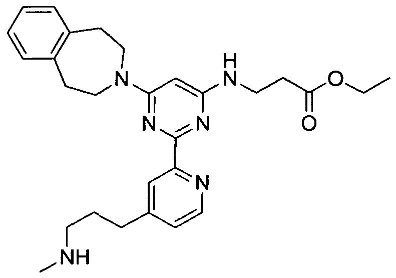

To a solution of ethyl *N*-[2-[4-(3-oxopropyl)-2-pyridinyl]-6-(1,2,4,5-tetrahydro-3*H*-3-benzazepin-3-yl)-4-pyrimidinyl]-β-alaninate (81 mg, 0.171 mmol) in THF (4 mL) was added MgSO₄ (515 mg, 4.28 mmol) and methanamine (0.428 mL, 0.855 mmol, 2M solution in THF). The mixture was stirred for 16 h and an aliquot removed. Sodium borohydride (6.47 mg, 0.171 mmol) was then added and the reaction mixture stirred at rt for 50 min. The reaction was quenched by the addition of water (20 mL) and DCM (20 mL). The layers were separated and the aqueous layer further extracted with DCM (2 x 20 mL). The combined organics were dried over Na₂SO₄ then concentrated *in vacuo* to afford the crude product as a yellow oil. The crude product was taken up in Ethanol (2 mL), HCl (1.0M in Et₂O) (0.2 mL, 0.200 mmol) was added and the reaction mixture allowed to stand for 1 h. A further portion of HCl (1.0M in Et₂O) (0.4 mL, 0.400 mmol) was added and the reaction mixture allowed to stand for a further 30 min. The reaction was concentrated in *vacuo* to afford the crude product as a yellow oil. The crude product was purified by column chromatography using a gradient of 0 to 100% of a 20% solution of 2M NH₃/MeOH in DCM)/DCM to give the title product as a yellow oil, 58 mg (69%).

LCMS (Method A): Rt = 0.74 min, MH+ = 489.4.

### Intermediate 10

### Ethyl N-{2-{4-[2-(1,3-dioxolan-2-yl)ethyl]-2-pyridinyl}-6-[(phenylmethyl)amino]-4-pyrimidinyl}-β-alaninate

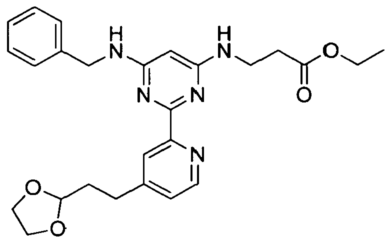

To a solution of ethyl *N*-(2-{4-[2-(1,3-dioxolan-2-yl)ethyl]-2-pyridinyl}-6-{[(4-methylphenyl)sulfonyl]oxy}-4-pyrimidinyl)-β-alaninate (285 mg, 0.525 mmol) in Isopropanol (10 mL) in a microwave vial was added 1-phenylmethanamine (0.287 mL, 2.63 mmol). The resultant solution was heated to 150 °C in a microwave for 1 h. The reaction mixture was heated at 150 °C for a further 1 hr. The reaction mixture was then diluted with EtOAc (10mL) and concentrated *in vacuo* to give the crude product as a yellow oil. The crude product was purified by silica chromatography using a gradient of 0 to 40% of a 20% solution of MeOH in DCM/DCM to give the impure product as a yellow oil. The product was further purified in two batches by MDAP (Method E).

In a separate reaction: to a solution of ethyl *N*-(2-{4-[2-(1,3-dioxolan-2-yl)ethyl]-2-pyridinyl}-6-{[(4-methylphenyl)sulfonyl]oxy}-4-pyrimidinyl)-β-alaninate (100 mg, 0.184 mmol) in Isopropanol (3 mL) in a 2-5mL microwave vial was added 1-phenylmethanamine (0.101 mL, 0.921 mmol). The resultant solution was heated in a microwave to 150 °C for 1 hr and then at 150 °C for a further 1 h. The reaction mixture was then diluted with EtOAc (10 mL) and concentrated *in vacuo* to give the crude product as a yellow oil. The crude product was purified by silica chromatography using a gradient of 0 to 40% of a 20% solution of MeOH in DCM/DCM to give the impure product as a yellow oil. Further purification was attempted using 0 to 100% of a 10% solution of MeOH in EtOAc/EtOAc. The same column was then eluted with 20 to 100% of a 20% solution of MeOH in DCM/DCM and finally the same column was eluted with 100% of a 20% solution of 2M NH₃ in MeOH/DCM. All of the fractions containing UV active material were concentrated together and the resultant crude material (~60mg) further purified by MDAP (Method E).

The appropriate fractions from *both* reactions were combined to afford the title compound as an off-white gum (96 mg, 28%).

LCMS (Method A): Rt = 0.92 min, MH+ = 478.3.

### Intermediate 11

### Ethyl N-{2-[4-(3-oxopropyl)-2-pyridinyl]-6-[(phenylmethyl)amino]-4-pyrimidinyl}-β-alaninate

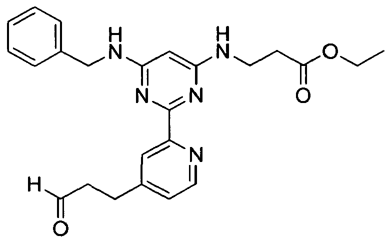

A solution of ethyl *N-*{2-{4-[2-(1,3-dioxolan-2-yl)ethyl]-2-pyridinyl}-6-[(phenylmethyl)amino]-4-pyrimidinyl}-β-alaninate (96 mg, 0.201 mmol) in Acetic Acid (3 mL)/Water (0.750 mL) was heated to 80 °C and stirred for 16 hr. The reaction mixture was diluted with dichloromethane (20 mL) and then quenched by the addition of NaHCO₃ solution. The quenched reaction was stirred until the effervescense ceased and the layers then separated. The aqueous layer was extracted with further DCM (2 x 20 mL) and the combined organics washed sequentially with NaHCO₃ solution (20 mL) and then brine (20 mL). The organics were dried over Na₂SO₄ then concentrated *in vacuo.* The crude product was re-dissolved in DCM (2 mL) and a small aliquot (~0.1mL) removed and dried under a stream of N₂ to allow an NMR to be taken. The remainder of the solution was concentrated to afford the crude title product as a yellow-green oil, 87 mg (100 %) which was used directly in the next step without further purification.

LCMS (Method B): Rt = 1.01 min, MH+ = 434.3.

### Intermediate 12

### Ethyl N-{2-{4-[3-(methylamino)propyl]-2-pyridinyl}-6-[(phenylmethyl)amino]-4-pyrimidinyl}-β-alaninate

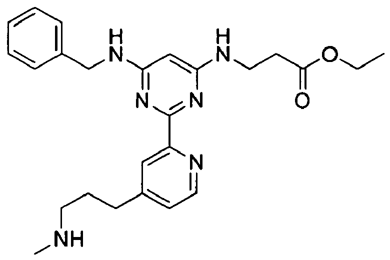

To a solution of ethyl *N-*{2-[4-(3-oxopropyl)-2-pyridinyl]-6-[(phenylmethyl)amino]-4-pyrimidinyl}-β-alaninate (42.5 mg, 0.098 mmol) in Tetrahydrofuran (THF) (2.3 mL) was added magnesium sulfate (295 mg, 2.451 mmol) and methanamine (0.245 mL, 0.490 mmol). The mixture was stirred for 16 h. Sodium borohydride (3.71 mg, 0.098 mmol) was then added and the reaction mixture stirred at rt for 45 min. The reaction was quenched by the addition of water (20 mL) and DCM (20 mL). The layers were separated and the aqueous layer further extracted with DCM (2 x 20 mL). The combined organics were concentrated *in vacuo* to afford the crude product as a yellow oil. The crude product was taken up in Ethanol (1 mL), HCl (1.0M in Et₂O) (0.3 mL, 0.300 mmol) was added and the reaction mixture allowed to stand for 1 h. The product was concentrated *in vacuo* to afford the crude product as a yellow oil. The crude product was purified by silica chromatography using a gradient of 0 to 100% of a 20% solution of 2M NH3 in MeOH/DCM to give the product as a yellow oil which still contained a ~10% impurity. The sample was further purified by MDAP (Method E) to give the title product as a colourless gum (12 mg, 27%)

LCMS (Method A): Rt = 0.67 min, MH+ = 449.3.

### Intermediate 13

### Ethyl N-{2-[4-(3-{[2-({[(1,1-dimethylethyl)oxy]carbonyl}amino)ethyl]amino}propyl)-2-pyridinyl]1-6-[(phenylmethyl)amino]-4-pyrimidinyl}-β-alaninate

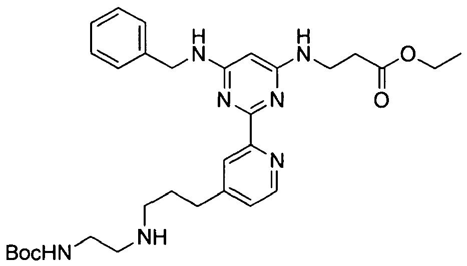

To a solution of ethyl *N*-{2-[4-(3-oxopropyl)-2-pyridinyl]-6-[(phenylmethyl)amino]-4-pyrimidinyl}-β-alaninate (42.5 mg, 0.098 mmol) in Tetrahydrofuran (THF) (2.3 mL) was added magnesium sulfate (295 mg, 2.451 mmol) and 1,1-dimethylethyl (2-aminoethyl)carbamate (79 mg, 0.490 mmol). The mixture was stirred for 16 h. Sodium borohydride (3.71 mg, 0.098 mmol) was added in 1 portion and the reaction stirred for 1 hr. The reaction was quenched by the addition of water (20 mL) and DCM (20 mL). The layers were separated and the aqueous layer further extracted with DCM (2 x 20 mL). The combined organics were dried (Na₂SO₄) and concentrated *in vacuo* to afford the crude product as a yellow oil. The crude product was taken up in Ethanol (1 mL) and HCl (1.0M in Et₂O) (0.3 mL, 0.300 mmol) added. The resultant solution was allowed to stand for 2 h and then concentrated *in vacuo* to afford the crude product as a yellow oil. The crude product was purified by silica chromatography using a gradient of 0 to 100% solution of 20% 2M NH₃/MeOH in DCM/DCM to give the impure product as a yellow oil. The product was further purified by MDAP (Method E). The appropriate fractions were combined together and concentrated *in vacuo* to afford the title product as a yellow gum, 19 mg (34%).

LCMS (Method A): Rt = 0.79 min, MH+ = 578.4.

### Intermediate 14

### Ethyl N-{2-(4-{3-[(2-aminoethyl)amino]propyl}-2-pyridinyl)-6-[(phenylmethyl)amino]-4-pyrimidinyl}-β-alaninate

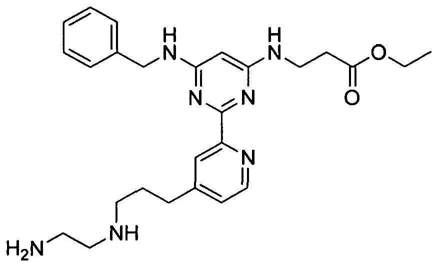

To a solution of ethyl *N*-{2-[4-(3-{[2-({[(1,1-dimethylethyl)oxy] carbonyl}amino)ethyl]amino}propyl)-2-pyridinyl]-6-[(phenylmethyl)amino]-4-pyrimidinyl}-β-alaninate (19 mg, 0.033 mmol) in Dichloromethane (DCM) (1 mL) at rt was added TFA (0.228 mL, 2.96 mmol) The mixture was stirred for 1 hr. The reaction mixture was concentrated under reduced pressure to afford a yellow oil which was then dissolved in methanol and loaded onto an SCX cartridge (5g) and eluted with methanol. Product was eluted with 2M ammonia in methanol. The filtrate from the ammonia fractions was concentrated under reduced pressure to yield the title compound as a yellow oil (15 mg, 95 %).

LCMS (Method B): Rt = 0.83 min, MH+ = 478.3.

### Intermediate 15

### 4-[(phenylamino)methyl]-2-pyridinecarbonitrile

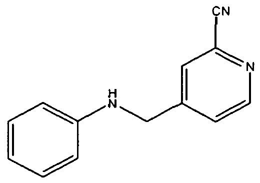

A mixture of 4-formyl-2-pyridinecarbonitrile (1.38 g, 10.45 mmol) and aniline (1.167 g, 12.53 mmol) in MeOH (200 mL) was stirred for 1 hr then NaCNBH₃ (3.28 g, 52.2 mmol) was added and the mixture stirred for 1hr. The solvent was evaporated under reduced pressure to give the crude product which was purified by column chromatography, eluting with a hexane and EtOAc solvent system to give the title compound, 1.78 g, (81 %).

LCMS (Method D): Rt 1.54min, MH+ 210.1

### Intermediate 16

### 4-[(phenylamino)methyl]-2-pyridinecarboximidamide hydrochloride

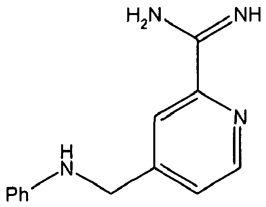

A mixture of 4-((phenylamino)methyl)picolinonitrile (1.78 g, 8.51 mmol) and sodium methoxide (0.092 g, 1.701 mmol) in methanol (100 mL) was stirred for 12 hr at rt. The solvent was evaporated in vacuo to give crude methyl 4-((phenylamino)methyl)picolinimidate (2 g). This was combined with ammonium chloride (0.887 g, 16.58 mmol) in Methanol (100 mL) and heated to 50 °C and stirred for 12 hr at this temperature. The solvent was evaporated in vacuo to give the crude product. The crude product was added to a silica gel column and was eluted with CH₂Cl₂/MeOH (10/1) to give 4-((phenylamino)methyl)picolinimidamide (1.67 g, 7.38 mmol)

¹H NMR (300 MHz, DMSO-*d*₆)ppm 9.58 (4H, br. s), 8.76 (1H,d), 8.40 (1H,s), 7.73 (1H, d), 7.09 (2H, t), 6.60-6.48 (4H, m), 4.44 (2H,d)

### Intermediate 17

### 6-hydroxy-2-{4-[(phenylamino)methyl]-2-pyridinyl}-4(1H)-pyrimidinone

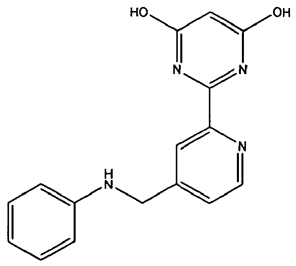

To a solution of sodium (0.848 g, 36.9 mmol) in Ethanol (150 mL) stirred under nitrogen at room temp was added a solution of 4-((phenylamino)methyl)picolinimidamide (1.67 g, 7.38 mmol) in Ethanol (50 mL) portionwise . The reaction mixture was stirred for 1 hr then diethyl propanedioate (2.364g, 14.8 mmol) was added portionwise. The reaction mixture was heated to reflux for 12 hr then allowed to cool to room temperature then acidified to pH 7 by adding concentrated HCl dropwise. The mixture was filtered and the filter cake was washed with water (100ml) then dissolved in MeOH (300 ml). The resulting mixture was filtered and the organic layer evaporated to give a solid (1.87g, 86%) which was used without any further purification

LCMS (Method D): Rt = 1.37 min, MH+ = 295.1

### Intermediate 18

### Phenylmethyl {[2-(4-hydroxy-6-oxo-1,6-dihydro-2-pyrimidinyl)-4-pyridinyl]methyl}phenylcarbamate

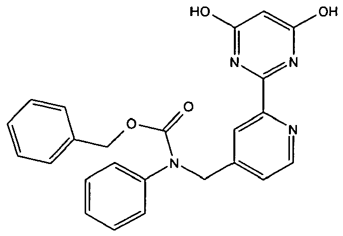

To a mixture of 6-hydroxy-2-{4-[(phenylamino)methyl]-2-pyridinyl}-4(1H)-pyrimidinone (100 mg, 0.340 mmol) and sodium hydrogencarbonate (143 mg, 1.699 mmol) in a solution of water (10 ml) and THF (10 ml) was added dropwise phenylmethyl chloridocarbonate (0.242 mL, 1.699 mmol).The mixture was stirred for 16 hr then concentrated under reduced pressure. The crude product was purified by column chromatography using MeOH in DCM to give the title compound, 93 mg (64 %).

LCMS (Method D): Rt 1.48 min, MH+ = 429.1

### Intermediate 19

### 6-hydroxy-2-{4-[(phenylamino)methyl]-2-pyridinyl}-4(1H)-pyrimidinone

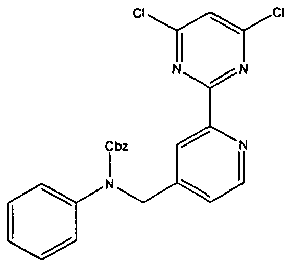

A mixture of phosphoric trichloride (20 mL) and benzyl ((2-(4,6-dihydroxypyrimidin-2-yl)pyridin-4-yl)methyl)(phenyl)carbamate (265 mg) was heated to reflux for 1 hr. The solvent was removed and crude product was purified by column chromatography using a hexane/EtOAc (5:1) solvent system to give the title compound (246 mg).

Rt 1.72 min, MH+ = 465.1 (Method D)

### Intermediate 20

### Methyl N-(6-chloro-2-{4-[(phenyl{[(phenylmethyl)oxy]carbonyl}amino)methyl]-2-pyridinyl}-4-pyrimidinyl)-β-alaninate

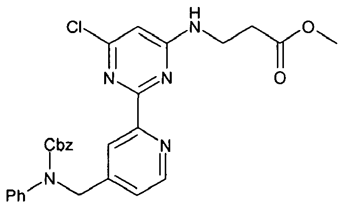

To a solution of benzyl ((2-(4,6-dichloropyrimidin-2-yl)pyridin-4-yl)methyl)(phenyl)carbamate (156 mg, 0.335 mmol) in 1,4-Dioxane (2 mL), was added DIPEA (0.176 mL, 1.006 mmol) and methyl β-alaninate hydrochloride (56.2 mg, 0.402 mmol) and the reaction heated in a CEM Discovery microwave at 100 °C for 2 h. After cooling the reaction mixture, the solvent was evaporated in vacuo to give a crude product which was added to a silica gel column and eluted with CH₂Cl₂/MeOH (20:1) to give the title compound (132 mg)

¹H NMR (300 MHz, DMSO-*d*₆) ppm 8.64 (1H, d), 8.20 (1H, s), 7.96 (1H, br s), 7.45-7.20 (11H, m), 6.60 (1H, s), 5.19 (2H, s), 5.06 (2H, s), 3.80-3.45 (5H, m), 2.67 (2H, t)

### Intermediate 21

### Ethyl N-[6-[(phenylmethyl)amino]-2-(2-pyridinyl)-4-pyrimidinyl]-β-alaninate

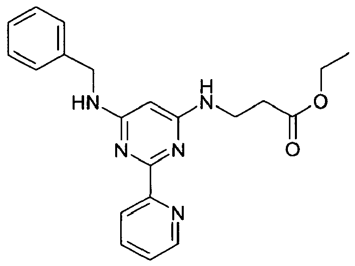

To a microwave vial with a stirrer was added ethyl *N*-[6-chloro-2-(2-pyridinyl)-4-pyrimidinyl]-β-alaninate (174 mg, 0.57 mmol), benzylamine (0.124 mL, 1.13 mmol) and DMSO (2 mL). The reaction vessel was sealed and heated using a microwave at 130 °C for 3 hr. The reaction mixture was allowed to cool, then material purified by MDAP. Appropriate fractions were combined and the solvent was evaporated under reduced pressure to give the title compound 57 mg (27%) as a pale yellow gum.

¹H NMR (400 MHz, DMSO-*d*₆) 8.64ppm (1H, d, CH), 8.18 (1H, d, CH), 7.85 (1H, ddd, CH), 7.44-7.18 (7H, m, CH, NH), 6.75 (1H, t, NH), 5.41 (1H, br. s, CH), 4.56-4.40 (2H, m, CH₂), 4.05 (2H, q, CH₂), 3.54-3.40 (2H, m, CH₂), 2.57 (2H, t, CH₂), 1.17 (3H, t, CH₃).

LCMS (Method A): Rt = 0.82min, MH⁺ 378

### Intermediate 22

### Ethyl N-[2-(2-pyridinyl)-6-(1,2,4,5-tetrahydro-3H-3-benzazepin-3-yl)-4-pyrimidinyl]-β-alaninate

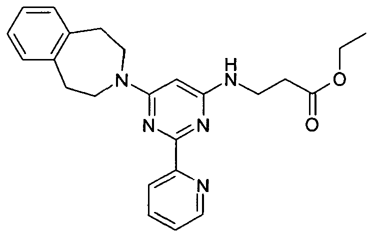

To a microwave vial with a stirrer was added 2,3,4,5-tetrahydro-1H-3-benzazepine (0.049 mL, 0.326 mmol), ethyl N-[6-chloro-2-(2-pyridinyl)-4-pyrimidinyl]-β-alaninate (100 mg, 0.326 mmol), DIPEA (0.114 mL, 0.652 mmol) and Dimethyl Sulfoxide (DMSO) (0.5 mL). The reaction vessel was sealed and heated in a Biotage Initiator microwave to 150 °C for 2.5 hr. After allowing the reaction mixture to cool, analysis by LCMS showed 73% conversion to the product. The reaction mixture was dissolved in DMSO to make the sample up to 1 mL and purified by MDAP. Product containing fractions were combined and the solvent evaporated in vacuo to give the title compound (99 mg) as a yellow oil.

LCMS (Method C): Rt = 1.22 min, MH⁺ 418

The intermediate indicated below was prepared similarly:

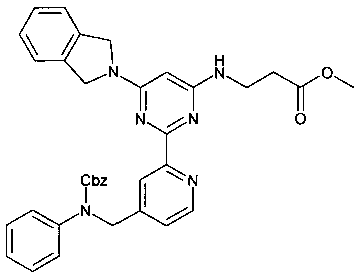

| **Intermediate** | **Yield /%** | **LCMS** |
|---|---|---|
| 23 *methyl N-(6-(1,3-dihydro-2H-isoindol-2-yl)-2-{4-[(phenylamino)methyl]-2-pyridinyl}-4-pyrimidinyl)-β-alaninate* | 68 | Rt = 1.46min, MH+=615.3, (Method D) |

### Example 1

### 3-((6-(4,5-dihydro-1H-benzo[d]azepin-3(2H)-yl)-2-(4-(3-(methylamino)propyl)pyridin-2-yl)pyrimidin-4-yl)amino)propanoic acid, Lithium salt

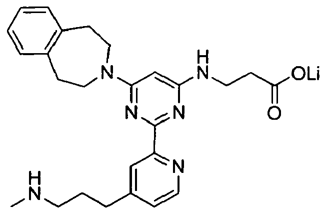

To a solution of ethyl 3-((6-(4,5-dihydro-1H-benzo[d]azepin-3(2H)-yl)-2-(4-(3-(methylamino)propyl)pyridin-2-yl)pyrimidin-4-yl)amino)propanoate (55 mg, 0.113 mmol) in Tetrahydrofuran (THF) (0.5 mL) / Water (0.500 mL) in a vial was added Lithium hydroxide monohydrate (4.72 mg, 0.113 mmol) and the reaction stirred for 30 min. Further Lithium hydroxide monohydrate (4.72 mg, 0.113 mmol) was added and the reaction stirred for a further 1 h in total. The solvent was removed under a stream of nitrogen and then dried in vacuo to afford the title compound as a yellow solid (51 mg, 0.109 mmol, 97 % yield).

LCMS (Method A): Rt = 0.63 min, MH⁺ 461

Other examples indicated in the following table were prepared similarly:

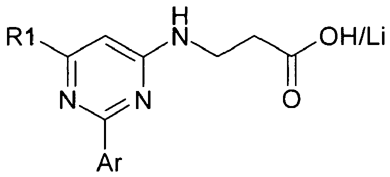

| **Example** | **R1** | **Ar** | **Yield %** | **LCMS** |
|---|---|---|---|---|
| 2 *Lithium N-{2-{4-[*3-*(methylamino)prop yl]-2-pyridinyl}-6-[(phenylmethyl)am ino]-4-pyrimidinyl}-β-alanine* | 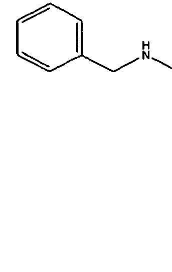 | 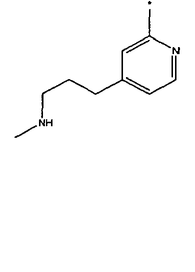 | Quantitative | Rt = 0.56 min, MH+ = 421.3 (Method A) |
| 3 *N-{2-(4-{3-[(2-aminoethyl)amino] propyl}-2-pyridinyl)-6-[(phenylmethyl)am ino]-4-pyrimidinyl}-β-alanine* | 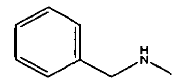 | 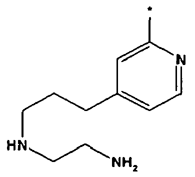 | 91 | Rt = 0.57 min, MH+ = 450.3 (Method B) |

### Example 4

### N-(6-(1,3-dihydro-2H-isoindol-2-yl)-2-{4-[(phenylamino)methyl]-2-pyridinyl}-4-pyrimidinyl)-β-alanine

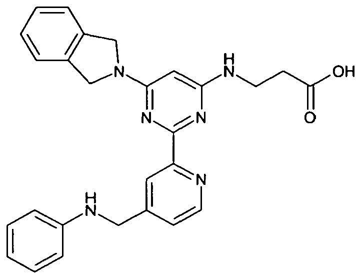

A mixture of methyl *N-*(6-(1,3-dihydro-2*H*-isoindol-2-yl)-2-{4-[(phenylamino)methyl]-2-pyridinyl}-4-pyrimidinyl)-β-alaninate (103 mg, 0.17 mmol) and KOH (40% by wt, 20ml) in MeOH (10 ml) was refluxed for 2h then material purified by prep-HPLC (Method F) to give the title compound as a solid, 46 mg.

LCMS (Method D) Rt 1.41min, MH+ = 467.2

The following Reference examples are not compounds used to form an immobilization product of the invention, but their use is referenced in the biological examples below.

### Reference Example 1

### N-[6-[(phenylmethyl)amino]-2-(2-pyridinyl)-4-pyrimidinyl]-β-alanine

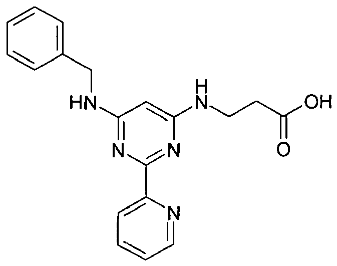

Ethyl N-[6-[(phenylmethyl)amino]-2-(2-pyridinyl)-4-pyrimidinyl]-β-alaninate (38 mg, 0.101 mmol) was dissolved in a 1:1:1 ratio of Methanol (0.3 mL), Tetrahydrofuran (THF) (0.300 mL) and Water (0.300 mL). To this was added lithium hydroxide (10 mg, 0.418 mmol) and stirred at room temperature for 4 hours. The reaction mixture was concentrated in vacuo and purified by MDAP. The appropriate fractions were combined, concentrated in vacuo and dried in a vacuum oven overnight to afford the title compound as a yellow solid (10 mg, 28.4% yield).

LCMS (Method B): Rt = 0.64min, MH⁺ 350

### Reference Example 2

### N-[2-(2-pyridinyl)-6-(1,2,4,5-tetrahydro-3H-3-benzazepin-3-yl)-4-pyrimidinyl]-β-alanine

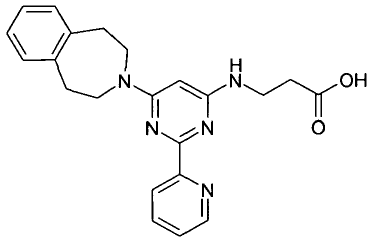

Ethyl N-[2-(2-pyridinyl)-6-(1,2,4,5-tetrahydro-3H-3-benzazepin-3-yl)-4-pyrimidinyl]-β-alaninate (135 mg, 0.323 mmol) was dissolved in a 1:1 ratio of Tetrahydrofuran (THF) (2 mL): Water (1 mL) . To this was added lithium hydroxide.monohydrate (13.57 mg, 0.323 mmol), the resulting mixture was stirred at room temperature for 16 hours. The solvent was removed under reduced pressure. The residues was dissolved in 1:1 MeOH:DMSO 2ml and purified by by prep-HPLC (Method E). The solvent was evaporated in vacuo to give the title compound as a yellow solid (Yield 90 mg).

LCMS (Method A): Rt = 0.80 min, MH⁺ 390

### Immobilization of compounds on beads (affinity matrix)

NHS-activated Sepharose 4 Fast Flow (Amersham Biosciences, 17-0906-01) was equilibrated with anhydrous DMSO (Dimethylsulfoxid, Fluka, 41648, H20 <= 0.005%). 1 ml of settled beads was placed in a 15 ml Falcon tube, compound stock solution (usually 100 mM in DMF or DMSO) was added (final concentration 0.2-2 µmol/ml beads) as well as 15 µl of triethylamine (Sigma, T-0886, 99% pure). Beads were incubated at room temperature in darkness on an end-over-end shaker (Roto Shake Genie, Scientific Industries Inc.) for 16 - 20 hours. Coupling efficiency is determined by HPLC. Non-reacted NHS-groups were blocked by incubation with aminoethanol at room temperature on the end-over-end shaker over night. Beads were washed with 10 ml of DMSO and were stored in isopropanol at -20°C. These beads were used as the affinity matrix in the following examples. Control beads (no compound immobilized) were generated by blocking the NHS-groups by incubation with aminoethanol as described above.

### Biological examples

### Bead assay using immobilized example 1 and HL-60 cells

### Principle of the assay

This example demonstrates the use of immobilized example 1 for the capturing and identification of histone demethylases from HL60 cell lysate in a competition binding assay. 100 µM of reference example 2 or DMSO was incubated with HL-60 cell lysate aliquots. Then the affinity matrix with the immobilized example 1 was added to capture proteins that were not interacting with the previously added reference compound. Beads were separated from the lysate and bead bound proteins were eluted in SDS sample buffer and subsequently separated by SDS-Polyacrylamide gel electrophoresis. The gel was stained with colloidal Coomassie and stained areas of each gel lane were cut out and subjected to in-gel proteolytic digestion with trypsin. Peptides originating from the different gel areas were labeled with isobaric tagging reagents (TMT reagents, Thermofisher) as shown in Table 2. The TMT reagents are a set of multiplexed, amine-specific, stable isotope reagents that can label peptides in up to six different biological samples enabling simultaneous identification and quantitation of peptides. The combined samples were fractionated using reversed-phase chromatography at pH 11 and fractions were subsequently analyzed with a nano-flow liquid chromatography system coupled online to a tandem mass spectrometer (LC-MS/MS) experiment followed by reporter ion quantification in the MS/MS spectra (Ross et al., 2004. Mol. Cell. Proteomics 3(12):1154-1169; Dayon et al., 2008. Anal. Chem. 80(8):2921-2931; Thompson et al., 2003. Anal. Chem. 75(8):1895-1904). Further experimental protocols can be found in WO2006/134056 and previous publications (Bantscheff et al., 2007. Nat. Biotechnol. 25, 1035-1044; Bantscheff et al., 2011. Nat. Biotechnol. 29(3):255-265).

### Results

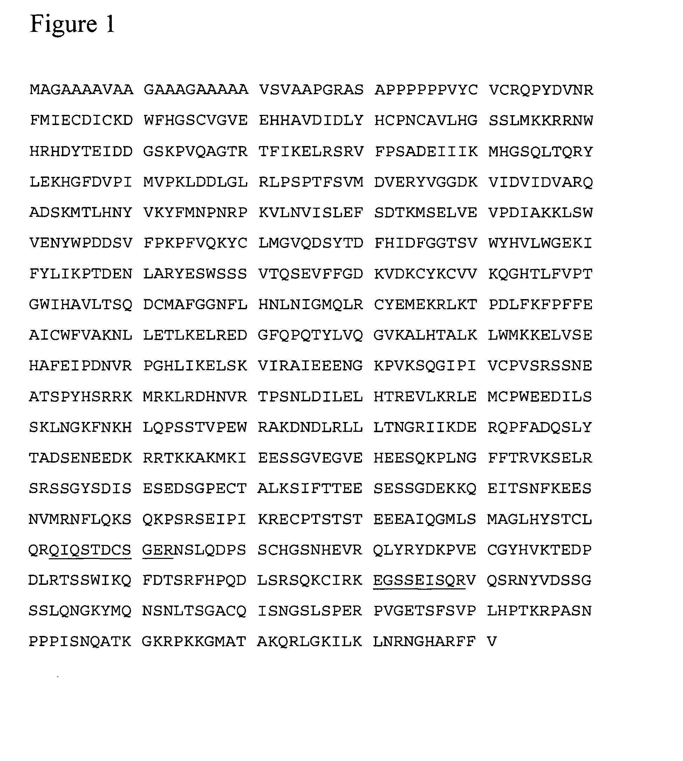

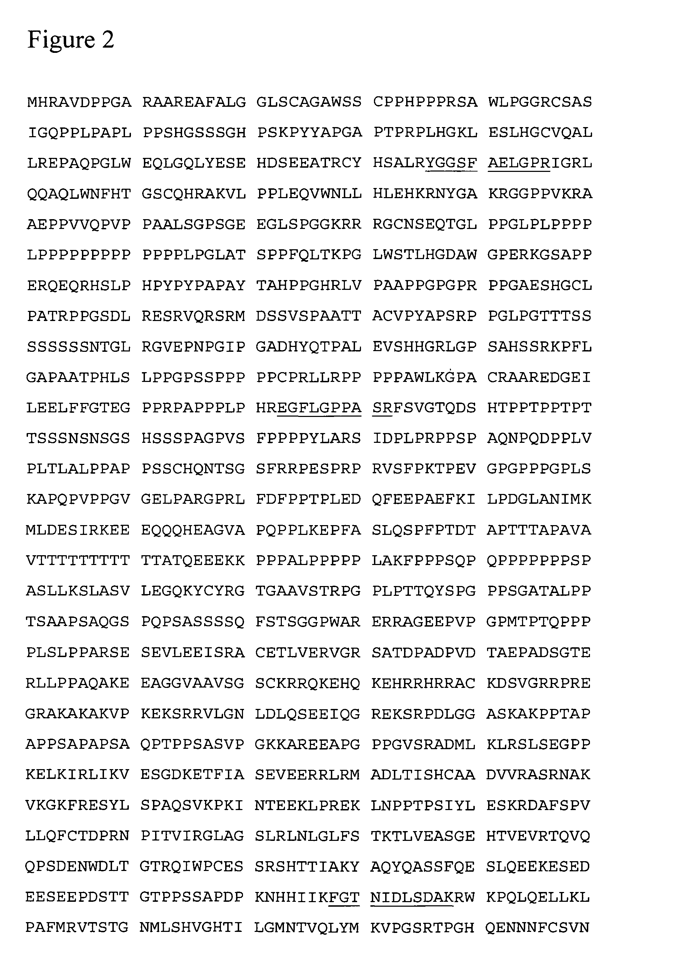

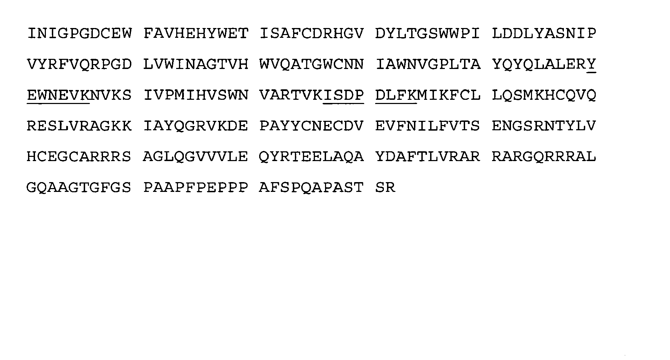

The identified JMJC domain histone demethylases are shown in Table 5 including the relative residual binding amount for each protein following competition with 100 µM of reference example 2 (relative residual binding of 0.10 corresponds to 90% of the protein being competed). Two different JMJC proteins were identified and competed by different degrees. Sequence identifiers are defined by the International Protein Index (IPI) (Kersey et al., 2004. Proteomics 4(7): 1985-1988). For illustration, the identified peptides for individual proteins are shown in Figures 1 and 2.

### Protocols

### 1. Cell culture

HL-60 cells (ATCC CCL-240, Manassas, VA, USA) were grown in spinner flasks (Integra Bioscience 182101, Zizers, Switzerland) in IMDM medium (Invitrogen 21980.065, Carlsbad, CA, USA) supplemented with 20% fetal calf serum (PAA Laboratories 15/101, Pasching, Austria) up to a cell concentration of 1x10⁶ cells/ml. Cells were treated for 16 hours with 80 nM of phorbol 12-myristate 13-acetate (PMA, Sigma, P1585). Cells were harvested by centrifugation and washed once with 1 x PBS buffer.

### 2. Preparation of cell lysate (nuclear extract)

Cell pellets were resuspended in 4 volumes of hypotonic buffer (10 mM TRIS-Cl, pH 7.4, 1.5 mM MgCl₂ (Sigma M-1028), 10 mM KCl , 25 mM NaF (Sigma S7920), 1 mM Na₃VO₄ (Sigma S6508). The cells were allowed to swell for 3 minutes (swelling checked under microscope) and then centrifuged for 5 minutes at 2,350 rpm (Heraeus). The supernatant was discarded and the pellet was resuspended in 2 volumes of hypotonic buffer supplemented with protease inhibitors. Cells were homogenized by 10 to 15 strokes in a homogenizer (VWR SCERSP885300-0015, Radnor, PA, USA) and the homogenate was centrifuged for 10 minutes at 3,300 rpm. The supernatant was discarded and the nuclei were washed in 3 volumes of hypotonic buffer supplemented with protease inhibitors (1 tablet for 25 ml; Roche 13137200, Basel, Switzerland) and centrifuged for 15 minutes at 10.000 rpm in a SLA-600TC rotor (Sorvall 74503). The pellet was resuspended in 1 volume of extraction buffer (50 mM TRIS-Cl, pH 7.4, 1.5 mM MgCl₂, 20 % glycerol (Merck Z835091), 420 mM NaCl (Sigma S5150), 25 mM NaF, 1 mM Na₃VO₄, 400 units/ml of DNAse I (Sigma D4527), and protease inhibitors (1 tablet for 25 ml)) and then homogenized first with 20 strokes with a homogenizer and then by 30 minutes gentle mixing at 4°C. The homogenate was then centrifuged for 30 minutes at 10,000 rpm in a SLA-600TC rotor. The supernatant was diluted in dilution buffer (1.8 ml buffer per 1 ml supernatant; 50 mM TRIS-Cl, pH 7.4, 1.5 mM MgCl₂, 25 mM NaF, 1 mM Na₃VO₄, 0.6 % Igepal CA-630 (Sigma, 13021) and protease inhibitors (1 tablet for 25 ml)). After 10 minutes incubation on ice, the lysate was centrifuged for 1 hour at 33,500 rpm in a Ti50.2 rotor (Beckman Coulter LE90K, 392052, Brea, CA, USA) and the supernatant was frozen in liquid nitrogen and stored at -80°C. After thawing of the nuclear lysate, the protein concentration was adjusted to 5 mg/ml. Sodium-L-ascorbate (Merck KGa, 1.03861) and FeCl₂ (Sigma, A4034) are added to the lysate at final concentration of 200 µM and 10 µM, respectively. The final buffer composition was 50 mM TRIS pH 7.4, 5% Glycerol, 150 mM NaCl, 25 mM NaF, 1.5 mM MgCl₂, 0.4% Igepal CA-630, 200 µM sodium-L-ascorbate, 10 µM FeCl₂ and protease inhibitors (1 tablet for 25 ml lysate). The lysate is then submitted to ultracentrifugation at 33,500 rpm for 20 minutes in a Ti50.2 rotor.

### 3. Capturing of histone demethylases from cell lysate

Sepharose-beads with the immobilized compound (35 µl beads per pull-down experiment) were equilibrated in lysis buffer and incubated with a HL-60 cell lysate sample containing 4.3 mg of protein on an end-over-end shaker (Roto Shake Genie, Scientific Industries Inc.) for 2 hours at 4°C. Beads were collected, transferred to Mobicol-columns (MoBiTech 10055) and washed with 10 ml lysis buffer containing 0.4% NP40 detergent, followed by 5 ml lysis buffer containing 0.2 % detergent. To elute bound proteins, 60 µl 2x SDS sample buffer containing 50 mM DTT was added to the column. The column was incubated for 30 minutes at 50°C and the eluate was transferred to a siliconized microfuge tube by centrifugation. Proteins were then alkylated with 108 mM iodoacetamide. Proteins were then separated by SDS-Polyacrylamide electrophoresis (SDS-PAGE).

### 4. Protein identification and quantitation by mass spectrometry

### 4.1 Protein digestion prior to mass spectrometric analysis

Gel-separated proteins were digested in-gel essentially following a previously described procedure (Shevchenko et al., 1996, Anal. Chem. 68:850-858). Briefly, gel-separated proteins were excised from the gel using a clean scalpel, destained twice using 100 µl 5mM triethylammonium bicarbonate buffer (TEAB; Sigma T7408) and 40% ethanol in water and dehydrated with absolute ethanol. Proteins were subsequently digested in-gel with porcine trypsin (Promega) at a protease concentration of 10 ng/µl in 5mM TEAB. Digestion was allowed to proceed for 4 hours at 37°C and the reaction was subsequently stopped using 5µl 5% formic acid. Gel plugs were extracted twice with 20 µl 1% formic acid and three times with increasing concentrations of acetonitrile. Peptide extracts were subsequently pooled with acidified digest supernatants and dried in a vacuum centrifuge.

### 4.2 TMT labeling of peptide extracts

The peptide extracts corresponding to the different aliquots treated with 100 µM of reference example 2 and the DMSO control were labeled with variants of the isobaric tagging reagent as shown in Table 2 (TMT sixplex Label Reagent Set, part number 90066, Thermo Fisher Scientific Inc., Rockford, IL 61105 USA). The TMT reagents are a set of multiplexed, amine-specific, stable isotope reagents that can label peptides on amino groups in up to six different biological samples enabling simultaneous identification and quantification of peptides. The TMT reagents were used according to instructions provided by the manufacturer. The samples were resuspended in 10 µl 50 mM TEAB solution, pH 8.5 and 10 µl acetonitrile were added. The TMT reagent was dissolved in acetonitrile to a final concentration of 24 mM and 10 µl of reagent solution were added to the sample. The labeling reaction was performed at room temperature for one hour on a horizontal shaker and stopped by adding 5 µl of 100 mM TEAB and 100 mM glycine in water. The labelled samples were then combined, dried in a vacuum centrifuge and resuspended in 60% 200mM TEAB / 40% acetonitrile. 2 µl of a 2.5% NH₂OH solution in water were added, incubated for 15 min and finally the reaction was stopped by addition of 10 µl of 20% formic acid in water. After freeze-drying samples were resuspended in 50 µl 0.1 % formic acid in water.

**Table 2: Labeling of peptides with TMT isobaric tagging reagents**

| **Gel lane** | **Sample** | **TMT6 reagent** |
|---|---|---|
| 1 | 100 µM reference example 2 | 128 |
| 2 | DMSO | 129 |

### 4.3. Peptide fractionation using high pH reversed phase chromatography

Peptide samples were injected into a capillary LC system (CapLC, Waters) and separated using a reversed phase C18 column (X-Bridge 1 mm x 150 mm, Waters, USA). Gradient elution was performed at a flow-rate of 50 µL/min. Solvent A: 20 mM ammoniumformiate, pH11, and solvent B: 20 mM ammoniumformiate, pH11, 60% acetonitrile and 1 min fractions were automatically collected throughout the separation range (Micro-fraction collector, Sunchrom, Germany) and pooled to yield a total of 16 peptide fractions.

**Table 3: Peptide fractionation by gradient elution**

| **Time (minutes)** | **% Solvent A** | **% Solvent B** |
|---|---|---|
| 0 | 95 | 5 |
| 5 | 95 | 5 |
| 90 | 55 | 45 |
| 90.2 | 15 | 85 |
| 92 | 15 | 85 |
| 99 | 15 | 85 |
| 99.2 | 95 | 5 |
| 110 | 95 | 5 |

### 4.4 LC-MS/MS analysis

Samples were dried *in vacuo* and resuspended in 0.1 % formic acid in water and aliquots of the sample were injected into a nano-LC system (Eksigent 1D+) coupled to LTQ-Orbitrap mass spectrometers (Thermo-Finnigan). Peptides were separated on custom 50 cm x 75uM (ID) reversed phase columns (Reprosil) at 40°C. Gradient elution was performed from 2% acetonitrile to 40% acetonitrile in 0.1 % formic acid over 2 hrs (Solvent A was 0.1 % formic acid and solvent B was 70% acetonitrile in 0.1% formic acid).

LTQ-Orbitrap XL and Orbitrap Velos instruments were operated with XCalibur 2.0/2.1 software. Intact peptides were detected in the Orbitrap at 30.000 resolution. Internal calibration was performed using the ion signal from (Si(CH₃)₂O)₆H⁺ at m/z 445.120025 (Olsen et al., 2005. Mol. Cell Proteomics 4, 2010-2021). Data dependent tandem mass spectra were generated for up to six peptide precursors using a combined CID/HCD approach (Kocher et al., 2009. J. Proteome Res. 8, 4743-4752). For CID up to 5000 ions (Orbitrap XL) or up to 3000 ions (Orbitrap Velos) were accumulated in the ion trap within a maximum ion accumulation time of 200 msec.

**Table 4: Peptide elution off the LC system**

| **Time (minutes)** | **% Solvent A** | **% Solvent B** |
|---|---|---|
| 0 | 95 | 5 |
| 85 | 40 | 60 |
| 88 | 10 | 90 |
| 93 | 10 | 90 |
| 93.5 | 95 | 5 |
| 120 | 95 | 5 |

### 4.5 Peptide and protein identification

Mascot™ 2.0 (Matrix Science) was used for protein identification using 10 ppm mass tolerance for peptide precursors and 0.8 Da (CID) tolerance for fragment ions. Carbamidomethylation of cysteine residues and TMT modification of lysine residues were set as fixed modifications and S,T,Y phosphorylation, methionine oxidation, N-terminal acetylation of proteins and TMT modification of peptide N-termini were set as variable modifications. The search database consisted of a customized version of the IPI protein sequence database combined with a decoy version of this database created using a script supplied by Matrix Science (Elias et al., 2005. Nat. Methods 2, 667-675). Protein identifications were accepted as follows: i) For single spectrum to sequence assignments, we required this assignment to be the best match *and* a minimum Mascot score of 31 *and* a 10x difference of this assignment over the next best assignment. Based on these criteria, the decoy search results indicated <1% false discovery rate (FDR); ii) For multiple spectrum to sequence assignments and using the same parameters, the decoy search results indicate <0.1 % false discovery rate. For protein quantification a minimum of 2 sequence assignments matching to unique peptides was required. FDR for quantified proteins was <<0.1%.

### 4.6 Peptide and protein quantification

Centroided TMT reporter ion signals were computed by the XCalibur software operating and extracted from MS data files using customized scripts. Only peptides unique for identified proteins were used for relative protein quantification. Further spectra used for quantification were filtered according to the following criteria: Mascot ion score > 15, signal to background ratio of the precursor ion > 4, s2i > 0.5 (Savitski et al., 2010. J. Am. Soc. Mass Spectrom. 21(10):1668-79). Reporter ion intensities were multiplied with the ion accumulation time yielding an area value proportional to the number of reporter ions present in the mass analyzer. For compound competition binding experiments fold changes are reported based on reporter ion areas in comparison to vehicle control and were calculated using sum-based bootstrap algorithm. Fold changes were corrected for isotope purity as described and adjusted for interference caused by co-eluting nearly isobaric peaks as estimated by the s2i measure (Savitski et al., 2010. J. Am. Soc. Mass Spectrom. 21(10):1668-79).

**Table 5: Identified histone demethylases with immobilized example 1 from HL-60 and competition with reference example 2.**

| **Representative** | **Protein name** | **Target class** | **Quantified spectra** | **Reference example 2** (relative residual binding) | **DMSO** |
|---|---|---|---|---|---|
| IPI00418567.4 | JHDM1D | Fe2OG-JMJC | 3 | 0.28 | 1 |
| IPI00550090.7 | JMJD3 | Fe2OG-JMJC | 8 | 0.32 | 1 |

### Bead assay using immobilized example 1 and concentration response curve for reference example 2

### Principle of the assay

This example demonstrates the use of immobilized example 1 for the capturing and identification of histone demethylases from cell lysate in a competition binding assay using reference example 2 at decreasing concentrations (Table 6). Dilutions of reference example 2 (sample 1 to 5) or the same amount of DMSO (sample 6) was incubated with the lysate. Then the affinity matrix with the immobilized example 1 was added to capture proteins that were not interacting with the previously added free reference compound. Beads were separated from the lysate and bead bound proteins were eluted in SDS sample buffer and subsequently separated by SDS-Polyacrylamide gel electrophoresis. The gel was stained with colloidal Coomassie and stained areas of each gel lane were cut out and subjected to in-gel proteolytic digestion with trypsin. Peptides originating from the different gel areas were labeled with isobaric tagging reagents (TMT reagents, Thermofisher) as shown in Table 6.

### Results

The histone demethylase UTY and JMJD3 were specifically bound to immobilized example 1 beads and IC₅₀ and pIC₅₀ inhibition values for competition by reference example 2 are indicated in Table 9.

### Protocols

### 1. Construction of Flag-JMJD3(1142-1682) and transfection into HEK293 cells

JMJD3 DNA encoding the catalytic domain (residues 1141-1682, lacking residues 1637-1675; GenBank BC009994) was amplified by PCR and sub cloned into pCDNA3.1 (+) (Invitrogen, V79020) using *BamHI* and *EcoRI* A Flag tag was included in the 5' primer, downstream of the *BamHI* cloning site. The missing amino acids (1637-1675) were reintroduced by three successive PCR reactions, and the completed C-terminal fragment was subcloned into the pCDNA3.1-JMJD3 1141-1682 (1637-1675 deletion) plasmid using AfeI and Xhol restriction enzymes. A demethylase inactive variant was created by Quickchange mutagenesis (Agilent, 200521), resulting in a Histidine1390 to Alanine (H1390A) conversion.

HEK-293 cells (ATCC CRL-1573) were grown in 15 cm diameter plates (BD Bioscience, 353025) to subconfluency in DMEM medium (Invitrogen, 41965-039) supplemented with 10% Foetal Bovine Serum. HEK-293 cells were transfected with the Flag-JMJD2D expressing pTT5 plasmid using the Calcium phosphate precipitation technique (Graham FL, van der Eb AJ (1973). "A new technique for the assay of infectivity of human adenovirus 5 DNA". Virology 52 (2): 456-67.) Cells should be at 60% confluency. For one 15 cm diameter plate, 25 µg of plasmid was mixed with 250 µl of 2.5 M CaCl₂ (Merck 1.02382) and 975 µl of water. 1,250 µl of 2x BBS (50 mM BES (N-N-Bis (2-hydroxyethyl)-2-amino-ethanesulfonic acid), Sigma B4554; 280 mM NaCl, Merck 1.06404; 1.5 mM Na₂HPO4 Merck 1.06580, pH 6.95) was added drop wise to the solution with gentle mixing. After 10 minutes the solution was added drop wise to the cell culture plate. The next day, medium was changed and the following day cells were harvested by scrapping and washed once in 1 x PBS.

### 2. Preparation of cell lysate

HEK293 cells were homogenized in a Potter S homogenizer in lysis buffer: 50 mM Tris-HCl, 0.8% NP40, 5% glycerol, 150 mM NaCl, 1.5 mM MgCl₂, 25 mM NaF, 1 mM sodium vanadate, 1 mM DTT, pH 7.5. One complete EDTA-free tablet (protease inhibitor cocktail, Roche Diagnostics, 1 873 580) per 25 ml buffer was added. The material was dounced 20 times using a mechanized POTTER S, transferred to 50 ml falcon tubes, incubated for 30 minutes rotating at 4° C and spun down for 10 minutes at 20,000 x g at 4°C (10,000 rpm in Sorvall SLA600, precooled). The supernatant was transferred to an ultracentrifuge (UZ)-polycarbonate tube (Beckmann, 355654) and spun for 1 hour at 145.000 x g at 4°C (40.000 rpm in Ti50.2, precooled). The supernatant was transferred again to a fresh 50 ml falcon tube, the protein concentration was determined by a Bradford assay (BioRad) and samples containing 50 mg of protein per aliquot were prepared. The samples were immediately used for experiments or frozen in liquid nitrogen and stored frozen at -80°C. Nuclear extracts of THP-1 cells were prepared and mixed at a 1:1 ratio (volume/volume) with the HEK cell extract.

### 3. Capturing of histone demethylases from cell lysate

Lysate samples containing 5 mg of proteins were first incubated with reference example 2 dissolved in DMSO at different concentrations or DMSO alone (Table 6) on an end-over-end shaker (Roto Shake Genie, Scientific Industries Inc.) for 45 minutes at 4°C. Sepharose-beads with the immobilized example 1 (35 µl beads per pull-down experiment) were equilibrated in lysis buffer and then added to lysate samples for two hours at 4°C. Beads were collected, transferred to Mobicol-columns (MoBiTech 10055) and washed with 10 ml lysis buffer containing 0.4% NP40 detergent, followed by 5 ml lysis buffer containing 0.2 % detergent. To elute bound proteins, 60 µl 2x SDS sample buffer containing 50 mM DTT was added to the column. The column was incubated for 30 minutes at 50°C and the eluate was transferred to a siliconized microfuge tube by centrifugation. Proteins were then alkylated with 108 mM iodoacetamide. Proteins were then separated by SDS-Polyacrylamide electrophoresis (SDS-PAGE).

### 4. Protein identification and quantitation by mass spectrometry

### 4.1 Protein digestion prior to mass spectrometric analysis

Gel-separated proteins were digested in-gel essentially following a previously described procedure (Shevchenko et al., 1996, Anal. Chem. 68:850-858). Briefly, gel-separated proteins were excised from the gel using a clean scalpel, destained twice using 100 µl 5mM triethylammonium bicarbonate buffer (TEAB; Sigma T7408) and 40% ethanol in water and dehydrated with absolute ethanol. Proteins were subsequently digested in-gel with porcine trypsin (Promega) at a protease concentration of 10 ng/µl in 5mM TEAB. Digestion was allowed to proceed for 4 hours at 37°C and the reaction was subsequently stopped using 5µl 5% formic acid. Gel plugs were extracted twice with 20 µl 1% formic acid and three times with increasing concentrations of acetonitrile. Peptide extracts were subsequently pooled with acidified digest supernatants and dried in a vacuum centrifuge.

### 4.2 TMT labeling of peptide extracts

The peptide extracts corresponding to the different aliquots treated with different concentrations of reference example 2 were labeled with variants of the isobaric tagging reagent as shown in Table 6 (TMT sixplex Label Reagent Set, part number 90066, Thermo Fisher Scientific Inc., Rockford, IL 61105 USA). The TMT reagents are a set of multiplexed, amine-specific, stable isotope reagents that can label peptides on amino groups in up to six different biological samples enabling simultaneous identification and quantification of peptides. The TMT reagents were used according to instructions provided by the manufacturer. The samples were resuspended in 10 µl 50 mM TEAB solution, pH 8.5 and 10 µl acetonitrile were added. The TMT reagent was dissolved in acetonitrile to a final concentration of 24 mM and 10 µl of reagent solution were added to the sample. The labeling reaction was performed at room temperature for one hour on a horizontal shaker and stopped by adding 5 µl of 100 mM TEAB and 100 mM glycine in water. The labelled samples were then combined, dried in a vacuum centrifuge and resuspended in 60% 200mM TEAB / 40% acetonitrile. 2 µl of a 2.5% NH₂OH solution in water were added, incubated for 15 min and finally the reaction was stopped by addition of 10 µl of 20% formic acid in water. After freeze-drying samples were resuspended in 50 µl 0.1% formic acid in water.

**Table 6: Labeling of peptides with TMT isobaric tagging reagents**

| **Gel lane** | **Sample** | **TMT6 reagent** |
|---|---|---|
| 1 | 40 µM reference example 2 | 126 |
| 2 | 10 µM reference example 2 | 127 |
| 3 | 2.5 µM reference example 2 | 128 |
| 3 | 0.625 µM reference example 2 | 129 |
| 5 | 0.156 µM reference example 2 | 130 |
| 6 | DMSO | 131 |

### 4.3. Peptide fractionation using high pH reversed phase chromatography

Peptide samples were injected into a capillary LC system (CapLC, Waters) and separated using a reversed phase C18 column (X-Bridge 1 mm x 150 mm, Waters, USA). Gradient elution was performed at a flow-rate of 50 µL/min. Solvent A: 20 mM ammoniumformiate, pH11, and solvent B: 20 mM ammoniumformiate, pH11, 60% acetonitrile and 1 min fractions were automatically collected throughout the separation range (Micro-fraction collector, Sunchrom, Germany) and pooled to yield a total of 16 peptide fractions.

**Table 7: Peptide fractionation by gradient elution**

| **Time (minutes)** | **% Solvent A** | **% Solvent B** |
|---|---|---|
| 0 | 95 | 5 |
| 5 | 95 | 5 |
| 90 | 55 | 45 |
| 90.2 | 15 | 85 |
| 92 | 15 | 85 |
| 99 | 15 | 85 |
| 99.2 | 95 | 5 |
| 110 | 95 | 5 |

### 4.4 LC-MS/MS analysis

Samples were dried *in vacuo* and resuspended in 0.1 % formic acid in water and aliquots of the sample were injected into a nano-LC system (Eksigent 1D+) coupled to LTQ-Orbitrap mass spectrometers (Thermo-Finnigan). Peptides were separated on custom 50 cm x 75uM (ID) reversed phase columns (Reprosil) at 40°C. Gradient elution was performed from 2% acetonitrile to 40% acetonitrile in 0.1 % formic acid over 2 hrs (Solvent A was 0.1 % formic acid and solvent B was 70% acetonitrile in 0.1 % formic acid).

LTQ-Orbitrap XL and Orbitrap Velos instruments were operated with XCalibur 2.0/2.1 I software. Intact peptides were detected in the Orbitrap at 30.000 resolution. Internal calibration was performed using the ion signal from (Si(CH₃)₂O)₆H⁺ at m/z 445.120025 (Olsen et al., 2005. Mol. Cell Proteomics 4, 2010-2021). Data dependent tandem mass spectra were generated for up to six peptide precursors using a combined CID/HCD approach (Kocher et al., 2009. J. Proteome Res. 8, 4743-4752). For CID up to 5000 ions (Orbitrap XL) or up to 3000 ions (Orbitrap Velos) were accumulated in the ion trap within a maximum ion accumulation time of 200 msec.

**Table 8: Peptide elution off the LC system**

| **Time (minutes)** | **% Solvent A** | **% Solvent B** |
|---|---|---|
| 0 | 95 | 5 |
| 85 | 40 | 60 |
| 88 | 10 | 90 |
| 93 | 10 | 90 |
| 93.5 | 95 | 5 |
| 120 | 95 | 5 |

### 4.5 Peptide and protein identification

Mascot™ 2.0 (Matrix Science) was used for protein identification using 10 ppm mass tolerance for peptide precursors and 0.8 Da (CID) tolerance for fragment ions. Carbamidomethylation of cysteine residues and TMT modification of lysine residues were set as fixed modifications and S,T,Y phosphorylation, methionine oxidation, N-terminal acetylation of proteins and TMT modification of peptide N-termini were set as variable modifications. The search database consisted of a customized version of the IPI protein sequence database combined with a decoy version of this database created using a script supplied by Matrix Science (Elias et al., 2005. Nat. Methods 2, 667-675). Protein identifications were accepted as follows: i) For single spectrum to sequence assignments, we required this assignment to be the best match *and* a minimum Mascot score of 31 *and* a 10x difference of this assignment over the next best assignment. Based on these criteria, the decoy search results indicated <1% false discovery rate (FDR); ii) For multiple spectrum to sequence assignments and using the same parameters, the decoy search results indicate <0.1% false discovery rate. For protein quantification a minimum of 2 sequence assignments matching to unique peptides was required. FDR for quantified proteins was <<0.1%.

### 4.6 Peptide and protein quantification

Centroided TMT reporter ion signals were computed by the XCalibur software operating and extracted from MS data files using customized scripts. Only peptides unique for identified proteins were used for relative protein quantification. Further spectra used for quantification were filtered according to the following criteria: Mascot ion score > 15, signal to background ratio of the precursor ion > 4, s2i > 0.5 (Savitski et al., 2010. J. Am. Soc. Mass Spectrom. 21(10):1668-79). Reporter ion intensities were multiplied with the ion accumulation time yielding an area value proportional to the number of reporter ions present in the mass analyzer. For compound competition binding experiments fold changes are reported based on reporter ion areas in comparison to vehicle control and were calculated using sum-based bootstrap algorithm. Fold changes were corrected for isotope purity as described and adjusted for interference caused by co-eluting nearly isobaric peaks as estimated by the s2i measure (Savitski et al., 2010. J. Am. Soc. Mass Spectrom. 21(10):1668-79).

**Table 9: Identification of histone demethylases with immobilized example 1 and competition with different concentrations of reference example 2 to determine concentration-response curves and IC₅₀ values.**

| **Representative sequence identifier** | **Protein name** | **Target class** | **Quantified spectra** | **Reference example 2 IC50** | **Reference example 2 pIC50** |
|---|---|---|---|---|---|
| IPI00023013.3 | UTY | Fe2OG-JMJC | 21 | 1.4 µM | 5.9 |
| IPI00550090.7 | JMJD3 | Fe2OG-JMJC | 152 | 1.2 µM | 5.9 |

SEQUENCE LISTING

<110>  Cellzome AG

<120>  Immobilization products and methods for the identification of
       histone demethylase interacting molecules and for the
       purification of histone demethylase proteins

<130>  CEL67888EP

<160>  3

<170>  PatentIn version 3.5

<210>  1
<211>  941
<212>  PRT
<213>  Homo sapiens

<400>  1

Met Ala Gly Ala Ala Ala Ala Val Ala Ala Gly Ala Ala Ala Gly Ala
1               5                   10                  15

Ala Ala Ala Ala Val Ser Val Ala Ala Pro Gly Arg Ala Ser Ala Pro
            20                  25                  30

Pro Pro Pro Pro Pro Val Tyr Cys Val Cys Arg Gln Pro Tyr Asp Val
        35                  40                  45

Asn Arg Phe Met Ile Glu Cys Asp Ile Cys Lys Asp Trp Phe His Gly
    50                  55                  60

Ser Cys Val Gly Val Glu Glu His His Ala Val Asp Ile Asp Leu Tyr
65                  70                  75                  80

His Cys Pro Asn Cys Ala Val Leu His Gly Ser Ser Leu Met Lys Lys
                85                  90                  95

Arg Arg Asn Trp His Arg His Asp Tyr Thr Glu Ile Asp Asp Gly Ser
            100                 105                 110

Lys Pro Val Gln Ala Gly Thr Arg Thr Phe Ile Lys Glu Leu Arg Ser
        115                 120                 125

Arg Val Phe Pro Ser Ala Asp Glu Ile Ile Ile Lys Met His Gly Ser
    130                 135                 140

Gln Leu Thr Gln Arg Tyr Leu Glu Lys His Gly Phe Asp Val Pro Ile
145                 150                 155                 160

Met Val Pro Lys Leu Asp Asp Leu Gly Leu Arg Leu Pro Ser Pro Thr
                165                 170                 175

Phe Ser Val Met Asp Val Glu Arg Tyr Val Gly Gly Asp Lys Val Ile
            180                 185                 190

Asp Val Ile Asp Val Ala Arg Gln Ala Asp Ser Lys Met Thr Leu His
        195                 200                 205

Asn Tyr Val Lys Tyr Phe Met Asn Pro Asn Arg Pro Lys Val Leu Asn
    210                 215                 220

Val Ile Ser Leu Glu Phe Ser Asp Thr Lys Met Ser Glu Leu Val Glu
225                 230                 235                 240

Val Pro Asp Ile Ala Lys Lys Leu Ser Trp Val Glu Asn Tyr Trp Pro
                245                 250                 255

Asp Asp Ser Val Phe Pro Lys Pro Phe Val Gln Lys Tyr Cys Leu Met
            260                 265                 270

Gly Val Gln Asp Ser Tyr Thr Asp Phe His Ile Asp Phe Gly Gly Thr
        275                 280                 285

Ser Val Trp Tyr His Val Leu Trp Gly Glu Lys Ile Phe Tyr Leu Ile
    290                 295                 300

Lys Pro Thr Asp Glu Asn Leu Ala Arg Tyr Glu Ser Trp Ser Ser Ser
305                 310                 315                 320

Val Thr Gln Ser Glu Val Phe Phe Gly Asp Lys Val Asp Lys Cys Tyr
                325                 330                 335

Lys Cys Val Val Lys Gln Gly His Thr Leu Phe Val Pro Thr Gly Trp
            340                 345                 350

Ile His Ala Val Leu Thr Ser Gln Asp Cys Met Ala Phe Gly Gly Asn
        355                 360                 365

Phe Leu His Asn Leu Asn Ile Gly Met Gln Leu Arg Cys Tyr Glu Met
    370                 375                 380

Glu Lys Arg Leu Lys Thr Pro Asp Leu Phe Lys Phe Pro Phe Phe Glu
385                 390                 395                 400

Ala Ile Cys Trp Phe Val Ala Lys Asn Leu Leu Glu Thr Leu Lys Glu
                405                 410                 415

Leu Arg Glu Asp Gly Phe Gln Pro Gln Thr Tyr Leu Val Gln Gly Val
            420                 425                 430

Lys Ala Leu His Thr Ala Leu Lys Leu Trp Met Lys Lys Glu Leu Val

435                 440                 445

Ser Glu His Ala Phe Glu Ile Pro Asp Asn Val Arg Pro Gly His Leu
    450                 455                 460

Ile Lys Glu Leu Ser Lys Val Ile Arg Ala Ile Glu Glu Glu Asn Gly
465                 470                 475                 480

Lys Pro Val Lys Ser Gln Gly Ile Pro Ile Val Cys Pro Val Ser Arg
                485                 490                 495

Ser Ser Asn Glu Ala Thr Ser Pro Tyr His Ser Arg Arg Lys Met Arg
            500                 505                 510

Lys Leu Arg Asp His Asn Val Arg Thr Pro Ser Asn Leu Asp Ile Leu
        515                 520                 525

Glu Leu His Thr Arg Glu Val Leu Lys Arg Leu Glu Met Cys Pro Trp
    530                 535                 540

Glu Glu Asp Ile Leu Ser Ser Lys Leu Asn Gly Lys Phe Asn Lys His
545                 550                 555                 560

Leu Gln Pro Ser Ser Thr Val Pro Glu Trp Arg Ala Lys Asp Asn Asp
                565                 570                 575

Leu Arg Leu Leu Leu Thr Asn Gly Arg Ile Ile Lys Asp Glu Arg Gln
            580                 585                 590

Pro Phe Ala Asp Gln Ser Leu Tyr Thr Ala Asp Ser Glu Asn Glu Glu
        595                 600                 605

Asp Lys Arg Arg Thr Lys Lys Ala Lys Met Lys Ile Glu Glu Ser Ser
    610                 615                 620

Gly Val Glu Gly Val Glu His Glu Glu Ser Gln Lys Pro Leu Asn Gly
625                 630                 635                 640

Phe Phe Thr Arg Val Lys Ser Glu Leu Arg Ser Arg Ser Ser Gly Tyr
                645                 650                 655

Ser Asp Ile Ser Glu Ser Glu Asp Ser Gly Pro Glu Cys Thr Ala Leu
            660                 665                 670

Lys Ser Ile Phe Thr Thr Glu Glu Ser Glu Ser Ser Gly Asp Glu Lys
        675                 680                 685

Lys Gln Glu Ile Thr Ser Asn Phe Lys Glu Glu Ser Asn Val Met Arg
    690                 695                 700

Asn Phe Leu Gln Lys Ser Gln Lys Pro Ser Arg Ser Glu Ile Pro Ile
705                 710                 715                 720

Lys Arg Glu Cys Pro Thr Ser Thr Ser Thr Glu Glu Glu Ala Ile Gln
                725                 730                 735

Gly Met Leu Ser Met Ala Gly Leu His Tyr Ser Thr Cys Leu Gln Arg
            740                 745                 750

Gln Ile Gln Ser Thr Asp Cys Ser Gly Glu Arg Asn Ser Leu Gln Asp
        755                 760                 765

Pro Ser Ser Cys His Gly Ser Asn His Glu Val Arg Gln Leu Tyr Arg
    770                 775                 780

Tyr Asp Lys Pro Val Glu Cys Gly Tyr His Val Lys Thr Glu Asp Pro
785                 790                 795                 800

Asp Leu Arg Thr Ser Ser Trp Ile Lys Gln Phe Asp Thr Ser Arg Phe
                805                 810                 815

His Pro Gln Asp Leu Ser Arg Ser Gln Lys Cys Ile Arg Lys Glu Gly
            820                 825                 830

Ser Ser Glu Ile Ser Gln Arg Val Gln Ser Arg Asn Tyr Val Asp Ser
        835                 840                 845

Ser Gly Ser Ser Leu Gln Asn Gly Lys Tyr Met Gln Asn Ser Asn Leu
    850                 855                 860

Thr Ser Gly Ala Cys Gln Ile Ser Asn Gly Ser Leu Ser Pro Glu Arg
865                 870                 875                 880

Pro Val Gly Glu Thr Ser Phe Ser Val Pro Leu His Pro Thr Lys Arg
                885                 890                 895

Pro Ala Ser Asn Pro Pro Pro Ile Ser Asn Gln Ala Thr Lys Gly Lys
            900                 905                 910

Arg Pro Lys Lys Gly Met Ala Thr Ala Lys Gln Arg Leu Gly Lys Ile
        915                 920                 925

Leu Lys Leu Asn Arg Asn Gly His Ala Arg Phe Phe Val
    930                 935                 940

<210>  2
<211>  1682
<212>  PRT

<213>  Homo sapiens

<400>  2

<210> 3
<211> 1347
<212> PRT
<213> Homo sapiens

<400> 3

## Claims

1. An immobilization product comprising a compound of formula (I) 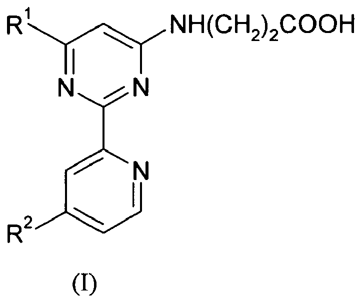 wherein
R¹ is:
C₃₋₇ cycloalkyl;
C₁₋₆ haloalkyl;
a 5, 6 or 7-membered aryl or heteroaryl (which heteroaryl contains one or more heteroatoms selected from N, O and S and which is optionally fused to phenyl),
said 5, 6 or 7-membered aryl or heteroaryl being optionally substituted with one or
more substituents independently selected from C₁₋₃alkyl;
O-C₁₋₆alkyl (which is optionally substituted by phenyl);
O- cyclohexyl (which is optionally fused with phenyl);
C(O)NR^{c}₂; or
NR^{a}R^{b},
each R^{a} and R^{b} is independently selected from:
H;
C₁₋₃alkyl which is optionally substituted by one or more substituents independently selected from phenyl (which phenyl is optionally substituted by one or more substituents independently selected from C₁₋₃alkyl, O-C₁₋₃alkyl, C(O)NR^{c}₂, halo and cyano), C(O)NR^{c}₂, a 4, 5, 6 or 7-membered heterocyclic or heteroaryl group (containing one or more heteroatoms independently selected from N, O and, S), a 3, 4, 5, 6 or 7-membered cycloalkyl group (which is optionally fused to phenyl), halo, OC₁₋₃alkyl, OH, -NHCOC₁₋₃alkylNR^{c}₂ and C(O)NHCH₂C(O)NR^{c}₂;
a 3, 4, 5, 6 or 7-membered cycloalkyl group (which is optionally fused to phenyl),
or
R^{a} and R^{b} together form a 5, 6 or 7-membered heterocyclic group optionally containing one or more further heteroatoms independently selected from N, O, S or S(O)₂ said heterocyclic group being optionally fused to a 5, 6 or 7-membered aryl or heteroaryl ring containing one or more heteroatoms independently selected from N, O and S; the heterocylic ring and/or the aryl or heteroaryl to which it is optionally fused being optionally substituted by one or more substituents independently selected from halo, OH, C₁₋₃alkyl, O-C₁₋₃alkyl, C(O)C₁₋₃alkyl, S(O)₂C₁₋₃alkyl, NHC(O)C₁₋₃alkyl, NHS(O)₂C₁₋₃alkyl, C(O)NR^{c}₂, C(O)NR^{d}₂ (wherein R^{d} and R^{d} together form a 5 or 6-membered heterocylic ring), NR^{c}₂, C(O)phenyl, S(O)₂NR^{c}₂, =O (oxo) and 5, 6 or 7-membered aryl or heteroaryl (containing one or more heteroatoms independently selected from N, O and S);
R² is:
(CH₂)₁₋₃NR^{c}(CH₂)₁₋₃NHR^{c},
(CH₂)₁₋₆NHR^{c};
(CH₂)₀₋₃NHR^{a} (wherein R^{a} is as defined above); or
(CH₂)₀₋₃NHPh;
and
each R^{c} is independently selected from hydrogen and C₁₋₃alkyl,
or a salt thereof;
**characterized in that** the compound of formula (I) is immobilized on a solid support.

2. The immobilization product of claim 1, wherein R¹ is NR^{a}R^{b}.

3. The immobilization product of any of claims 1 or 2, wherein
R¹ is NHCH₂Ph, 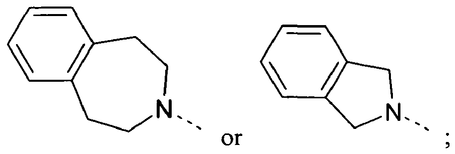 and
R² is (CH₂)₀₋₃NHPh or (CH₂)₁₋₆NHR^{c,}.

4. The immobilization product of claim 1, wherein the compound of formula (I) is selected from the group consisting of 3-((6-(4,5-dihydro-1H-benzo[d]azepin-3(2H)-yl)-2-(4-(3-(methylamino)propyl)pyridin-2-yl)pyrimidin-4-yl)amino)propanoic acid, N-{2-{4-[3-(methylamino)propyl]-2-pyridinyl}-6-[(phenylmethyl)amino]-4-pyrimidinyl}-β-alanine, N-{2-(4-{3-[(2-aminoethyl)amino]propyl}-2-pyridinyl)-6-[(phenylmethyl)amino]-4-pyrimidinyl}-β-alanine, and N-(6-(1,3-dihydro-2H-isoindol-2-yl)-2-{4-[(phenylamino)methyl]-2-pyridinyl}-4-pyrimidinyl)-β-alanine.

5. The immobilization product of any of claims 1 to 4, wherein the solid support is selected from the group consisting of agarose, modified agarose, sepharose beads (e.g. NHS-activated sepharose), latex, cellulose, and ferro- or ferrimagnetic particles.

6. The immobilization product of any of claims 1 to 5, wherein the immobilization product results from a covalent direct or linker mediated attachment of the immobilization compound to the solid support, in particular wherein the linker is a C₁₋₁₀ alkylene group, which is optionally interrupted or terminated by one or more atoms or functional groups selected from the group consisting of S, O, NH, C(O)O, OC(O), C(O), NHC(O), and C(O)NH and wherein the linker is optionally substituted with one or more substituents independently selected from the group consisting of halogen, OH, NH₂, C(O)H, C(O)NH₂, SO₃H, NO₂, and CN.

7. A method for the identification of a histone demethylase interacting compound, comprising the steps of
a) providing a protein preparation containing at least one histone demethylase,
b) contacting the protein preparation with the immobilization product of any of claims 1 to 6 and with a given compound under conditions allowing the formation of one or more different complexes between one of the histone demethylases and the immobilization product, and
c) detecting the complex or the complexes formed in step b).

8. A method for the identification of a histone demethylase interacting compound, comprising the steps of:
a) providing two aliquots of a protein preparation containing at least one histone demethylase,
b) contacting one aliquot with the immobilization product of any of claims 1 to 6 under conditions allowing the formation of one or more different complexes between one of the histone demethylases and the immobilization product,
c) contacting the other aliquot with the immobilization product of any of claims 1 to 6 and with a given compound under conditions allowing the formation of one or more different complexes between one of the histone demethylases and the immobilization product, and
d) determining the amount of the complex or the complexes formed in steps b) and c).

9. A method for the identification of a histone demethylase interacting compound, comprising the steps of:
a) providing two aliquots of a cell preparation comprising each at least one cell containing at least one histone demethylase,
b) incubating one aliquot with a given compound,
c) harvesting the cells of each aliquot,
d) lysing the cells in order to obtain protein preparations,
e) contacting the protein preparations with the immobilization product of any of claims 1 to 6 under conditions allowing the formation of one or more different complexes between one of the histone demethylases and the immobilization product, and
f) determining the amount of the complex or the complexes formed in each aliquot in step e).

10. The method of any of claims 8 to 9, wherein a reduced amount of the complex formed in the aliquot incubated with the compound in comparison to the aliquot not incubated with the compound indicates that said histone demethylase is a target of the compound.

11. The method of any of claims 8 to 10, wherein the amount of the complex is determined by separating the histone demethylase from the immobilization product and subsequent detection of the separated histone demethylase or subsequent determination of the amount of the separated histone demethylase, in particular wherein the histone demethylase is detected or the amount of the histone demethylase is determined by mass spectrometry or immunodetection methods, preferably with an antibody directed against the histone demethylase.

12. The method of any of claims 7 to 11, wherein said given compound is selected from the group consisting of synthetic compounds, or organic synthetic drugs, more preferably small molecule organic drugs, and natural small molecule compounds.

13. The method of any of claims 7 to 12, wherein the given compound is a histone demethylase inhibitor.

14. The method of any of claims 7 to 13, wherein the provision of a protein preparation includes the steps of harvesting at least one cell containing histone demethylases and lysing the cell.

15. The method of any of claims 7 to 14, wherein the steps of the formation of the complex are performed under essentially physiological conditions.

16. A method for determining the presence of one or more histone demethylases in a sample, comprising the steps of:
a) providing a protein preparation expected to contain said one or more histone demethylase,
b) contacting the protein preparation with the immobilization product of any of claims 1 to 6 under conditions allowing the formation of a complex between one of the histone demethylases and the immobilization product, and
c) detecting whether one or more histone demethylases have formed a complex with the immobilization product.

17. Use of the immobilization product according to any of claims 1 to 6 for the identification of histone demethylase interacting compounds or for the purification of histone demethylases.

18. The method of any of claims 7 to 16 or the use of claim 17, wherein the affinity of the histone demethylase interacting compound for the histone demethylase is determined.
